(19) Europäisches Patentamt
European Patent Office
Office européen des brevets

(11) Publication number: **0 409 565 A1**

(12) **EUROPEAN PATENT APPLICATION**

(21) Application number: **90307807.9**

(51) Int. Cl.[5]: **C08K 5/20**, C08L 21/00

(22) Date of filing: **17.07.90**

(30) Priority: **17.07.89 JP 185633/89**
**27.07.89 JP 196333/89**
**20.10.89 JP 273986/89**
**20.10.89 JP 273987/89**
**24.10.89 JP 277376/89**
**24.10.89 JP 277377/89**
**26.10.89 JP 281162/89**
**26.10.89 JP 281163/89**

(43) Date of publication of application:
**23.01.91 Bulletin 91/04**

(84) Designated Contracting States:
**DE FR GB IT**

(71) Applicant: **SUMITOMO CHEMICAL COMPANY, LIMITED**
**5-33, Kitahama 4-chome Chuo-ku**
**Osaka(JP)**

(72) Inventor: **Inui, Naoki**
**1598 Tsutsuicho**
**Yamatokoriyama-shi, Nara-ken(JP)**
Inventor: **Nagasaki, Hideo**
**4-7-7-Itakano, Higashiyodogawa-ku**
**Osaka-shi, Osaka-fu(JP)**
Inventor: **Yachigo, Shinichi**
**2-10-5-406 Sone Higasimachi**
**Toyonaka-shi, Osaka-fu(JP)**
Inventor: **Oikawa, Miyuki**
**1-2-40 Hirata**
**Ibaraki-shi, Osaka-fu(JP)**

(74) Representative: **Geering, Keith Edwin et al**
**REDDIE & GROSE 16 Theobalds Roadoad**
**London WC1X 8PL(GB)**

(54) **Rubber composition useful for tires.**

(57) A rubber composition comprising a natural and/or synthetic rubber, a filler and an acrylamide compound represented by the formula of

$$X-NH\underset{\underset{\displaystyle O}{\|}}{C}CH=CH_2$$

wherein X is an aliphatic or alicyclic group, phenyl or phenoxyphenyl, in which said aliphatic or alicyclic group may contain halogen or oxygen in the group, said phenyl may be substituted once or twice by halogen, hydroxy, alkyl of 1 to 8 carbon atoms, alkoxy of 1 to 8 carbon atoms, nitro, cyano, carboxy or alkoxycarbonyl, and said phenoxyphenyl is represented by the formula of

EP 0 409 565 A1

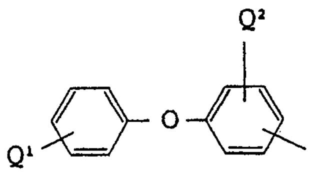

wherein $Q^1$ and $Q^2$ independently of one another are each hydrogen, alkyl of 1 to 8 carbon atoms, cyclohexyl, phenyl, halogen, hydroxy or amino. This rubber composition is increased in its loss factor, and is useful for tires and tread parts. The resulting tires prepared from the rubber composition are improved in their gripping performance.

## RUBBER COMPOSITION USEFUL FOR TIRES

The present invention relates to a rubber composition suitable for enhancing the gripping performance of automobile tires.

In recent years, the progress in high-performance automobiles, the extension of paved roads and the growth of superhighway networks have increased the demands to improve the gripping performance of tires which has close relation to acceleration and braking capability of automobiles. It is known that high gripping performance can be achieved if the energy loss by friction between tire tread and road surface is increased, and corresponding thereto, tread rubber materials having an elevated loss factor (tan $\delta$ ) on deformation are demanded.

Prior proposals to increase tan $\delta$ on deformation include, for example, the use of styrene/butadiene copolymer rubber of high styrene content as a base rubber, adding a large amount of process oil, filling the rubber with a large amount of highly reinforcing carbon black, and the like.

When styrene/butadiene copolymer rubber of high styrene content is used as the base rubber for tire treads, it gives good gripping performance up to a certain temperature region; but a high glass transition temperature (Tg) is a characteristic of such rubber and there is a great dependency of tan $\delta$ on temperature, and hence at elevated tire temperature (caused by driving on a hot road surface and/or by heat build-up from running) there is a rapid fall in tan $\delta$ and hence a decrease in gripping performance. When the base rubber is filled with a large amount of process oil or highly reinforcing carbon black, a rise in gripping performance is observed, but heat build-up in the rubber is increased and there is marked deterioration in the strength and abrasion resistance of the rubber.

We have intensively researched to increase the loss factor of tire rubber without decreasing its heat resistance while maintaining its strength characteristics at a high level, thereby enhancing gripping performance, especially when hot.

The present invention provides a rubber composition comprising natural and/or synthetic rubber, filler and acrylamide compound represented by the formula (I),

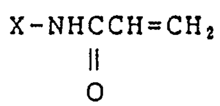

(I) ...

wherein X is an aliphatic, alicyclic, phenyl or phenoxyphenyl group, in which said aliphatic or alicyclic group may contain halogen or oxygen in the group, said phenyl may be substituted once or twice by substituent(s) selected from hydroxy, alkyl of 1 to 8 carbon atoms, alkoxy of 1 to 8 carbon atoms, nitro, cyano, carboxy and alkoxycarbonyl, and said phenoxyphenyl is represented by the formula (II)

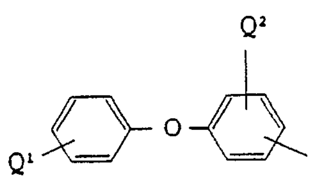

(II)

wherein $Q^1$ and $Q^2$ are selected independently from hydrogen, alkyl of 1 to 8 carbon atoms, cyclohexyl, phenyl, halogen, hydroxy and amino.

The present invention also provides a method for increasing the loss factor of a rubber which comprises blending the rubber with filler and acrylamide compound represented by the above formula (I).

The present invention further provides a novel acrylamide compound represented by the formula (III),

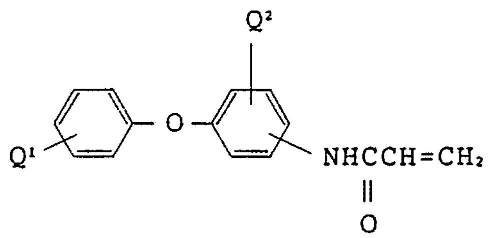

(III)

wherein $Q^1$ and $Q^2$ are selected independently from hydrogen, alkyl of 1 to 8 carbon atoms, cyclohexyl, phenyl, halogen, hydroxy and amino, which compound is one of the species of the formula (I).

The present invention still further provides a method for producing a compound represented by the above formula (III), which comprises subjecting phenoxyaniline compound represented by the formula (IV),

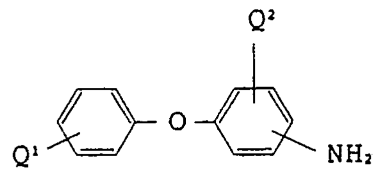

(IV)

wherein $Q^1$ and $Q^2$ are as defined above,
to a condensation reaction with acryloyl halide represented by the formula (V),

$$CH_2=CH\underset{\underset{\displaystyle O}{\|}}{C}-Z \qquad (V)$$

wherein Z is halogen,
in an inert solvent in the presence of dehydrohalogenating agent.

The compound represented by the above formula (I) can be prepared, in general, by subjecting a corresponding amine to a dehydrohalogenating reaction with an acryloyl halide in an innert solvent such as toluene, chloroform or the like, with the coexistence of a base compound such as triethylamine, pyridine, sodium hydroxide or the like.

The aliphatic group denoted by X in the formula (I) includes, for example, alkyl of 1 to 20 carbon atoms unsubstituted or substituted with halogen (e.g. fluorine, chlorine, bromine or iodine) or hydroxy, alkoxyalkyl of 2 to 20 total carbon atoms unsubstituted or substituted with halogen or hydroxy, and the like. The alicyclic group denoted by X in the formula (I) includes, for example, cyclohexyl unsubstituted or substituted with lower (e.g. $C_1$ to $C_4$ alkyl, halogen or hydroxy.

Phenyl denoted by X in the formula (I) may be unsubstituted or may be substituted once or twice by halogen, hydroxy, alkyl, alkoxy, nitro, cyano, carboxy or alkoxycarbonyl. Alkyl as the substituent for phenyl is of 1 to 8 carbon atoms, and may be cyclic or straight or branched, among which preferred is straight or branched alkyl of 1 to 4 carbon atoms. More preferred is methyl or ethyl. Alkoxy as the substituent for phenyl is of 1 to 8 carbon atoms, and may be branched when the carbon number is 3 or more, among which preferred is alkoxy of 1 to 4 carbon atoms, and more preferred is methoxy or ethoxy. The alkyl part of alkoxycarbonyl as the substituent for phenyl is, for example, of 1 to 4 carbon atoms, among which methyl and ethyl are preferred. Halogen as the substituent for phenyl includes, for example, fluorine, chlorine, bromine and iodine, in which preferred is chlorine or bromine.

When X in the formula (I) is phenoxyphenyl represented by the formula (II), the acrylamide compound of the formula (I) is represented by the formula (III). In the above formula (II) or (III), alkyl of 1 to 8 carbon atoms capable of constituting $Q^1$ or $Q^2$ includes methyl, ethyl, propyl, butyl, pentyl, hexyl, heptyl and octyl, and may be branched when its carbon number is 3 or more, besides the straight ones. Among them, preferred are those having relatively smaller carbon chains such as alkyl of 1 to 4 carbon atoms. Halogen capable of constituting $Q^1$ or $Q^2$ includes, for example, fluorine, chlorine, bromine and iodine, among which preferred are chlorine and bromine, and more preferred is chlorine. Amino capable of constituting $Q^1$ or $Q^2$ may be unsubstituted ($-NH_2$), or may be monosubstituted or disubstituted. Examples of the substituent for

amino are alkyl of 1 to 4 carbon atoms, phenyl, cyclohexyl and the like.

The preferred acrylamide compounds represented by the formula (I) applicable in the present invention are as follows:

A. Compounds of the formula (I) wherein X is an aliphatic or alicyclic group which may contain halogen or oxygen;

B. A compound of the formula (I) wherein X is unsubstituted phenyl;

C. Compounds represented by the formula (VI),

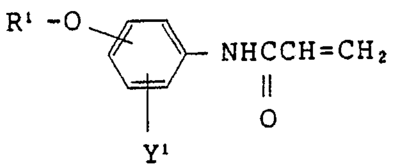

(VI)

wherein $R^1$ is hydrogen or alkyl, and $Y^1$ is hydrogen, halogen, hydroxy, alkoxy, cyano, carboxy or alkoxycarbonyl;

D. Compounds represented by the formula (VII),

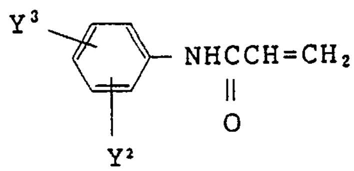

(VII)

wherein one of $Y^2$ and $Y^3$ is halogen, and the other is hydrogen, halogen, cyano, carboxy or alkoxycarbonyl;

E. Compounds represented by the formula (VIII),

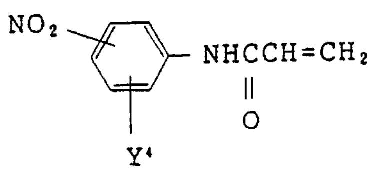

(VIII)

wherein $Y^4$ is hydrogen, alkyl, alkoxy, halogen, nitro, cyano, hydroxy, carboxy or alkoxycarbonyl;

F. Compounds represented by the formula (IX),

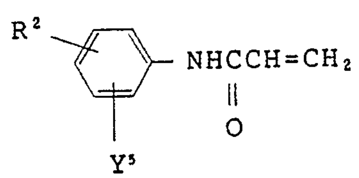

(IX)

wherein $R^2$ is alkyl, and $Y^5$ is hydrogen, alkyl, halogen, hydroxy, alkoxy, cyano, carboxy or alkoxycarbonyl;

G. Compounds represented by the formula (X),

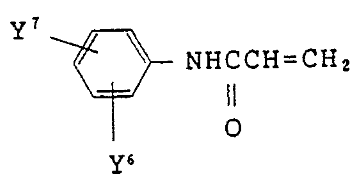

(X)

wherein one of $Y^6$ and $Y'$ is cyano, carboxy or alkoxycarbonyl, and the other is hydrogen, cyano, carboxy or alkoxycarbonyl; and

H. Compounds represented by the above formula (III).

The compounds of the above group A include, for example, the following :

(A-1) $CH_3-NHCOCH=CH_2$

(A-2) $CH_3CH_2-NHCOCH=CH_2$

(A-3) $CH_3(CH_2)_2-NHCOCH=CH_2$

(A-4) $CH_3(CH_2)_3-NHCOCH=CH_2$

(A-5) $CH_3(CH_2)_4-NHCOCH=CH_2$

(A-6) $CH_3(CH_2)_5-NHCOCH=CH_2$

(A-7) $CH_3(CH_2)_6-NHCOCH=CH_2$

(A-8) $CH_3(CH_2)_7-NHCOCH=CH_2$

(A-9) $CH_3(CH_2)_8-NHCOCH=CH_2$

(A-10) $CH_3(CH_2)_9-NHCOCH=CH_2$

(A-11) $CH_3(CH_2)_{11}-NHCOCH=CH_2$

(A-12) $CH_3(CH_2)_{17}-NHCOCH=CH_2$

(A-13) 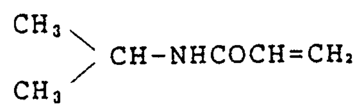

(A-14) 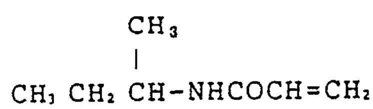

(A-15) 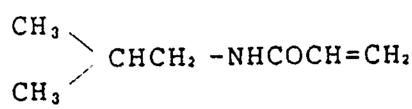

(A-16) 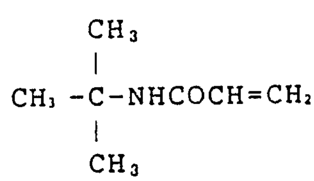

(A-17) 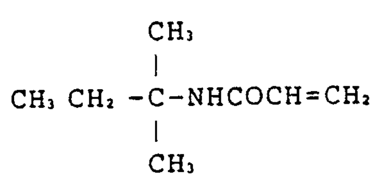

(A-18) 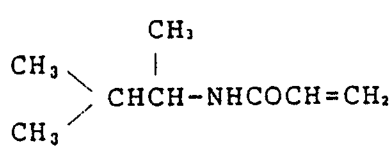

(A-19)

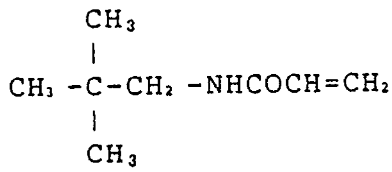

(A-20)

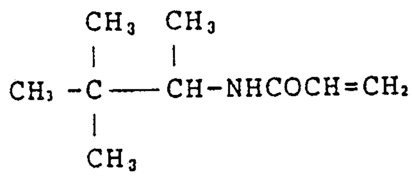

(A-21)

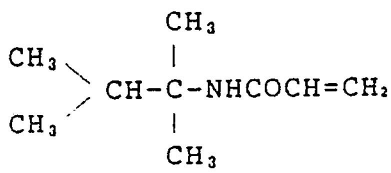

(A-22)

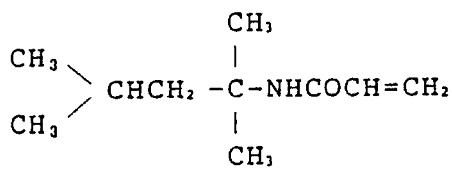

(A-23)

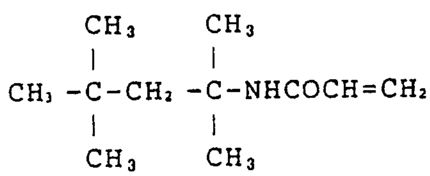

(A-24)

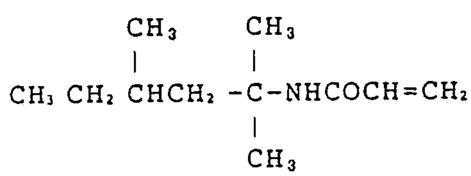

(A-25)

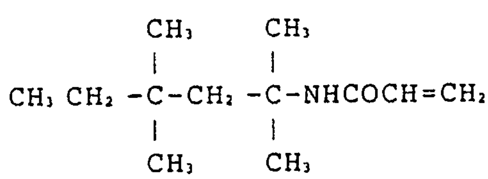

(A-26)

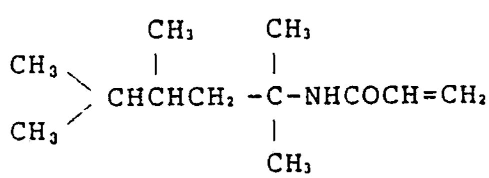

(A-27)

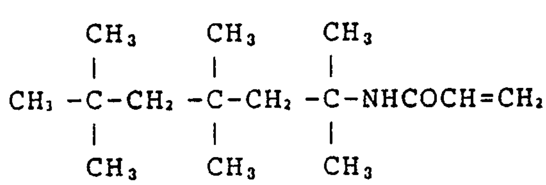

(A-28) 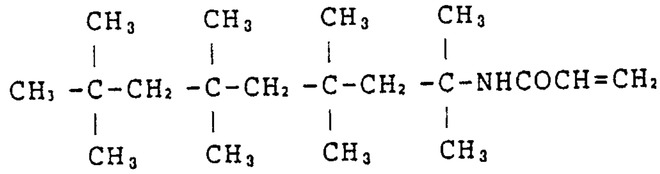

(A-29) 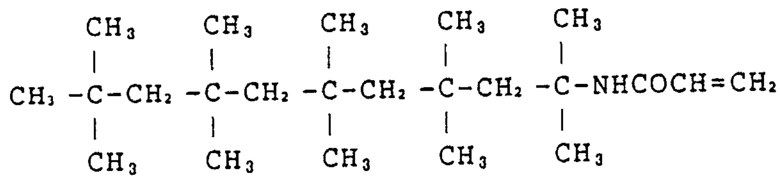

(A-30) 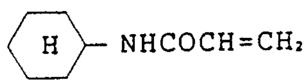

When X in the formula (I) is an aliphatic or alicyclic group, it may contain halogen or oxygen therein. Thus, the compounds of group A include, in addition to those exemplified above, those in which a part of the group corresponding to X in the formula (I) is substituted with halogen such as chlorine and bromine or with hydroxy, and those in which the group corresponding to X in the formula (I) has an ether linkage to form alkoxyalkyl.

The compounds of the formula (I) to be used in the present invention wherein X is an aliphatic or alicyclic group are not limited to those exemplifid above, but are preferably those in which X is an aliphatic group of 1 to 20 carbon atoms, more preferably those in which X is a branched aliphatic group of 3 to 20 carbon atoms. The aliphatic group denoted by X may of course be straight, but is preferably branched. Thus, the preferred compounds of the formula (I) in which X is an aliphatic or alicyclic group are those in which X is a branched aliphatic group, particularly alkyl, of 3 to 20 carbon atoms.

The compound of the above group B is N-phenylacrylamide represented by the formula of

(B) 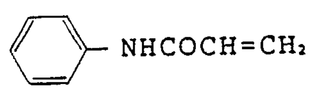 .

The compounds of the above group C represented by the formula (VI) include, for example, the following :

(C-1) 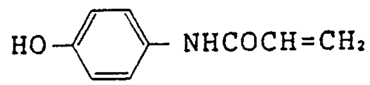

(C-2) 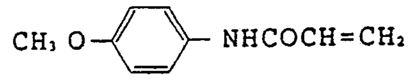

(C-3) 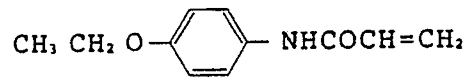

(C-4) 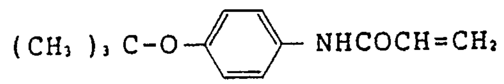

(C-5) 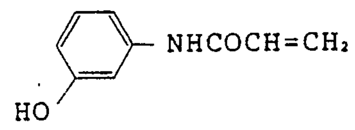

(C-6) 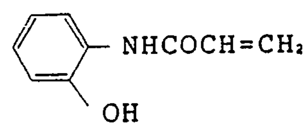

(C-7) 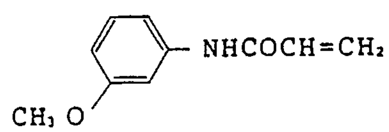

(C-8) 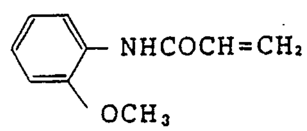

(C-9)

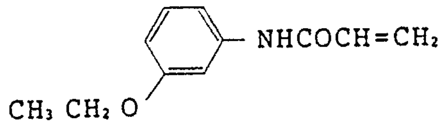

(C-10)

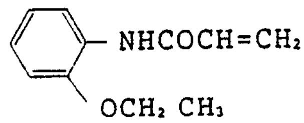

(C-11)

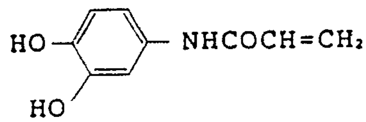

(C-12)

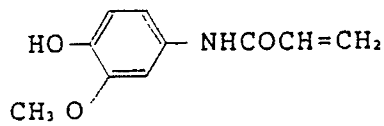

(C-13)

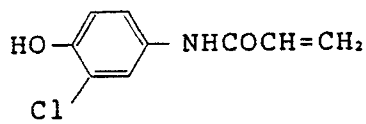

(C-14)

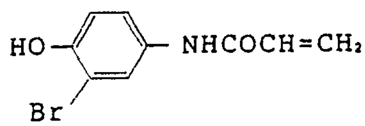

(C-15)

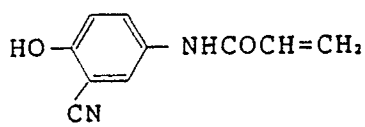

(C-16)

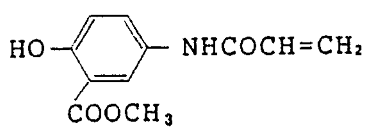

(C-17)

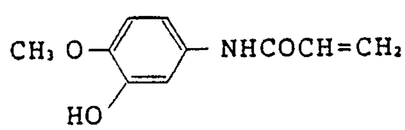

(C-18)

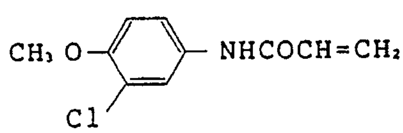

(C-19)

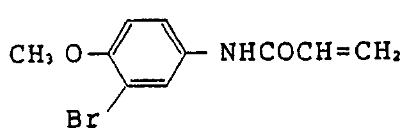

(C-20) 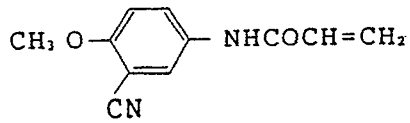

(C-21) 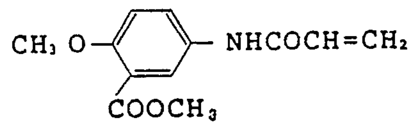

The compounds of the formula (VI) to be used in the present invention are not limited to those exemplified above, but are preferably those in which $Y^1$ is hydrogen. $R^1$-O- in the formula (VI) can be attached at any position of the benzene ring, but is preferably attached at 4-position of the benzene ring against the acryloylamino group.

The compounds of the above group D represented by the formula (VII) include, for example, the following :

(D-1) 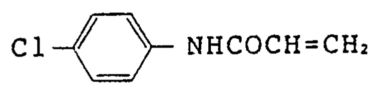

(D-2) 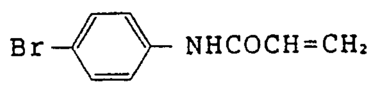

(D-3) 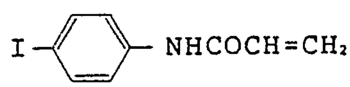

(D-4) 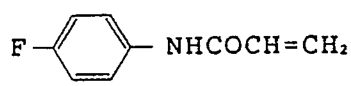

(D-5) 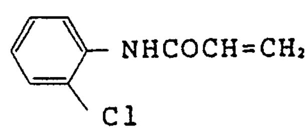

(D-6) 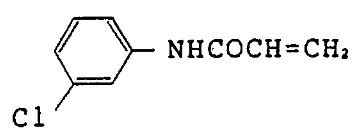

(D-7) 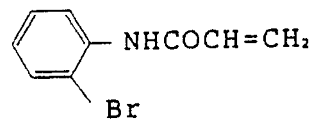

(D-8) 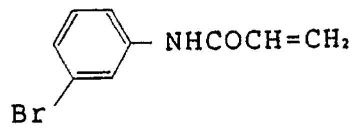

(D-9) 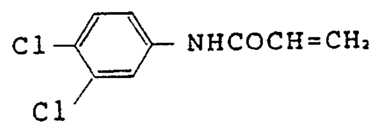

(D-10) 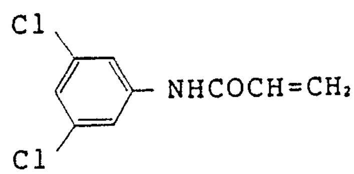

(D-11) 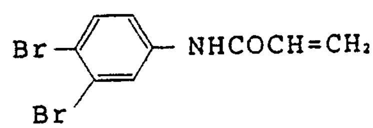

(D-12) 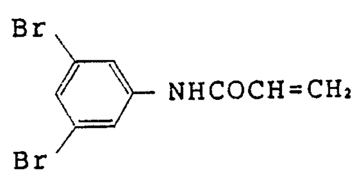

(D-13) 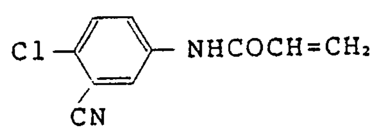

(D-14) 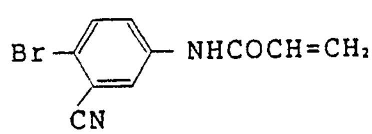

(D-15) 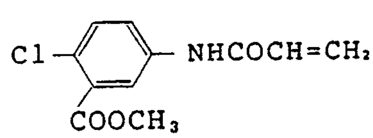

(D-16) 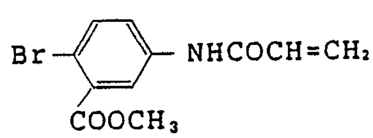

The compounds of the formula (VII) to be used in the present invention are not limited to those exemplified above, but are preferably those in which one of $Y_2$ and $Y_3$ is halogen and the other is hydrogen. Halogen denoted by $Y_2$ or $Y_3$ can be attached at any position of the benzene ring, but is preferably attached at 3- or 4-position of the benzene ring against the acryloylamino group.

The compounds of the above group E represented by the formula (VIII) include, for example, the following :

(E-1)

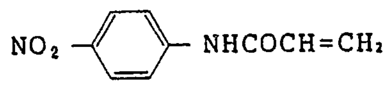

(E-2)

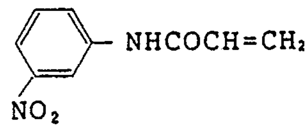

(E-3)

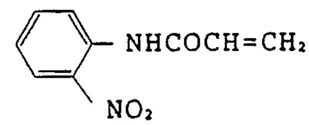

(E-4)

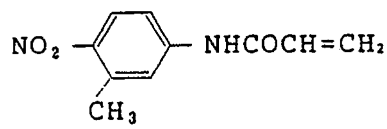

(E-5)

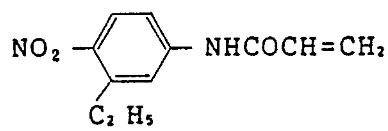

(E-6)

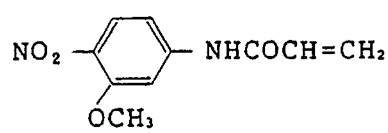

(E-7)

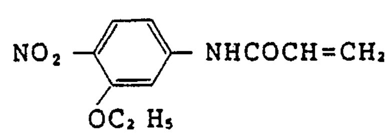

(E-8)

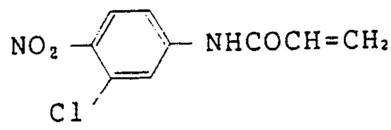

(E-9)

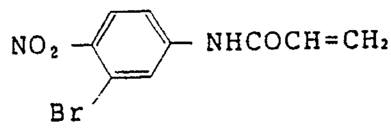

(E-10)

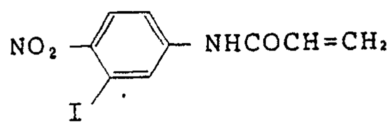

(E-11)

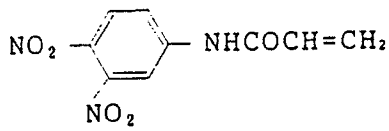

(E-12)

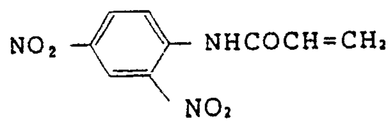

(E-13)

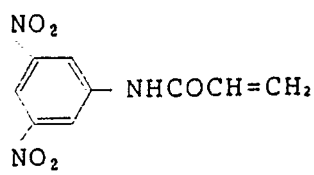

(E-14)

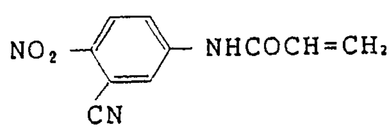

(E-15)

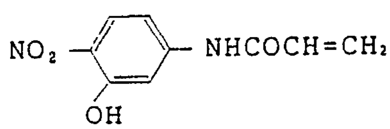

(E-16)

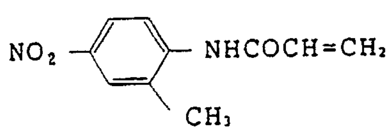

The compounds of the formula (VIII) to be used in the present invention are not limited to those exemplified above, but are preferably those in which $Y^4$ is hydrogen, alkoxy or nitro, and more preferably those in which $Y^4$ is hydrogen. Nitro in the formula (VIII) can be attached at any position of the benzene ring, but is preferably attached at 3- or 4-position of the benzene ring against the acryloylamino group.

The compounds of the above group F represented by the formula (IX) include, for example, the following :

(F-1) 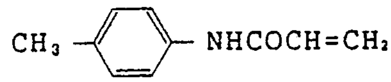

(F-2) 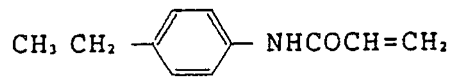

(F-3) 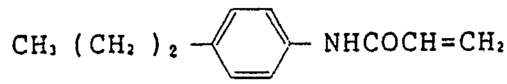

(F-4) 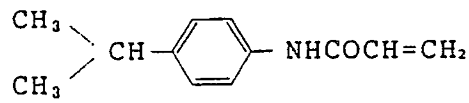

(F-5) 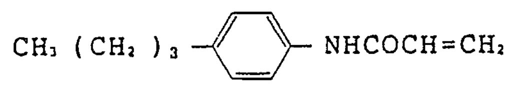

(F-6) 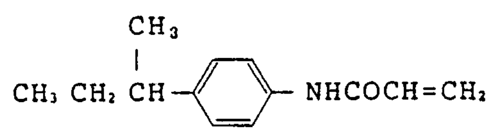

(F-7) 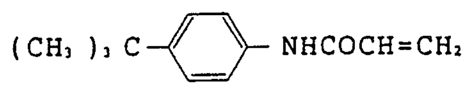

(F-8) 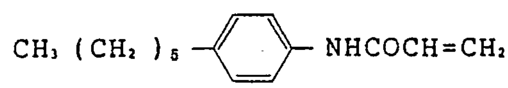

(F-9)

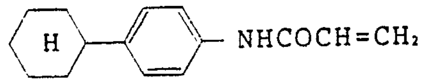

(F-10)

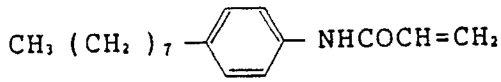

(F-11)

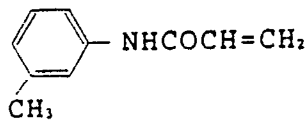

(F-12)

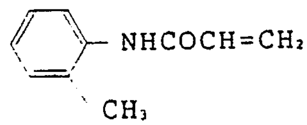

(F-13)

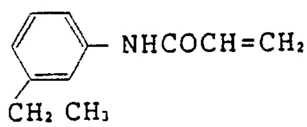

(F-14)

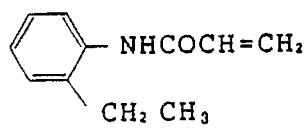

(F-15)

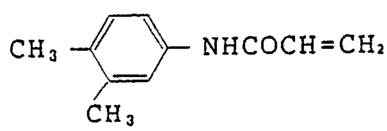

(F-16)

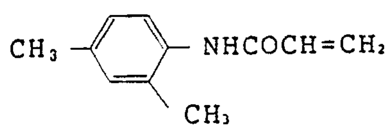

(F-17)

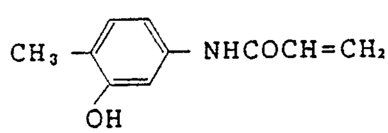

(F-18)

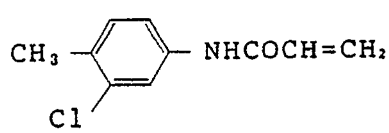

(F-19)

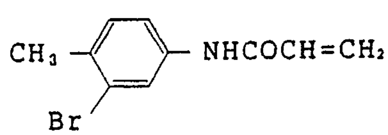

(F-20) 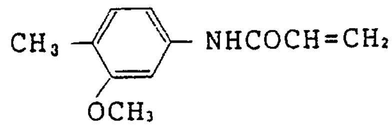

(F-21) 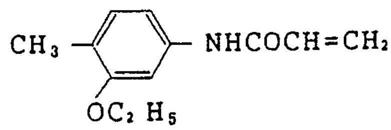

(F-22) 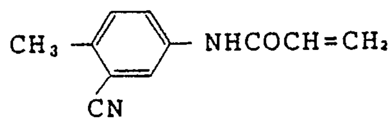

(F-23) 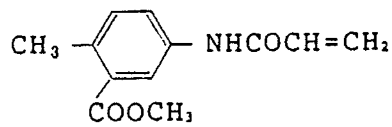

(F-24) 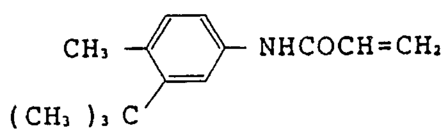

The compounds of the formula (IX) to be used in the present invention are not limited to those exemplified above, but are preferably those in which $Y^5$ is hydrogen. $R^2$ in the formula (IX) can be attached at any position of the benzene ring, but is preferably attached at 4-position of the benzene ring against the acryloylamino group. $R^2$ is preferably methyl or ethyl.

The compounds of the above group G represented by the formula (X) include, for example, the following :

(G-1) 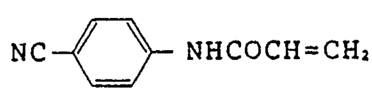

(G-2) 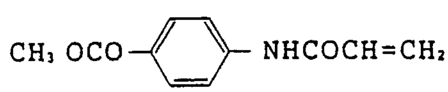

(G-3)

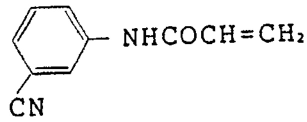

(G-4)

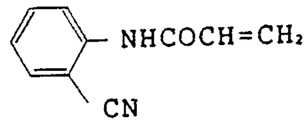

(G-5)

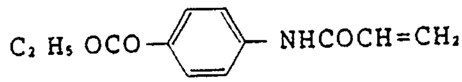

(G-6)

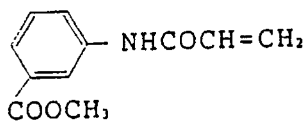

(G-7)

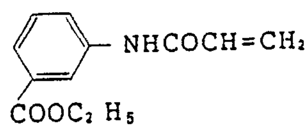

(G-8)

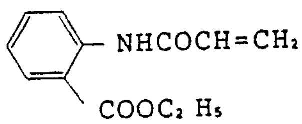

(G-9)

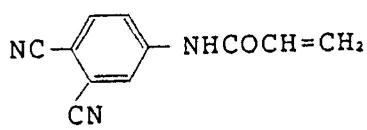

(G-10)

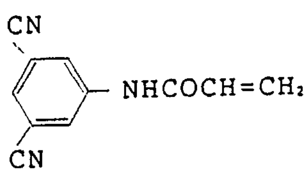

(G-11)

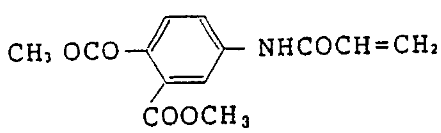

The compounds of the formula (X) to be used in the present invention are not limited to those exemplified above, but are preferably those in which one of $Y^6$ and $Y^7$ is cyano, carboxy or alkoxycarbonyl, and the other is hydrogen. N-(4-cyanophenyl)acrylamide is more preferred.

As to the compounds of the above group H represented by the formula (III), preferred $Q^1$ and $Q^2$ are, selected independently from hydrogen, alkyl of 1 to 4 carbon atoms, chlorine or unsubstituted or substituted amino.

More preferred compounds are those in which $Q^1$ is hydrogen, alkyl of 1 to 4 carbon atoms, chlorine or amino unsubstituted or substituted by alkyl of 1 to 4 carbon atoms, and $Q^2$ is hydrogen or alkyl of 1 to 4 carbon atoms.

When $Q^1$ is other than hydrogen, it can be bonded at any position of the benzene nucleus, but is preferably bonded at 4- or 3-position against the position of the etheric oxygen. When $Q^2$ is other than

hydrogen, it can also be bonded at any position of the benzene nucleus, but is preferably bonded at 2- or 3-position against the position of the etheric oxygen.

Specific examples of the compounds represented by the above formula (III) are illustrated below :

(H-1)

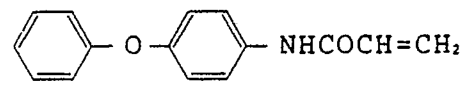

(H-2)

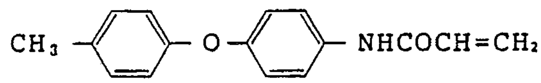

(H-3)

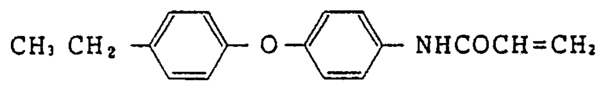

(H-4)

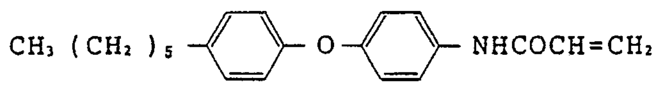

(H-5)

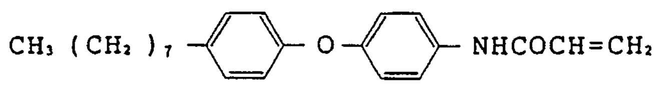

(H-6)

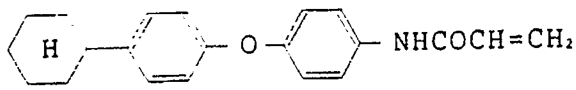

(H-7)

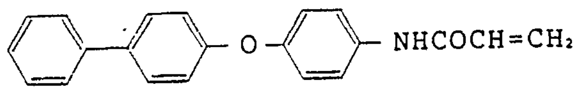

(H-8)

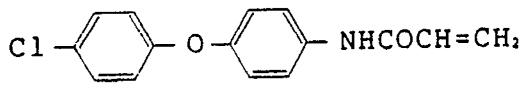

(H-9)

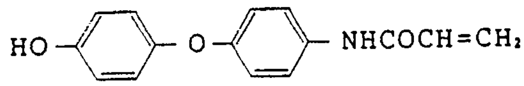

(H-10)

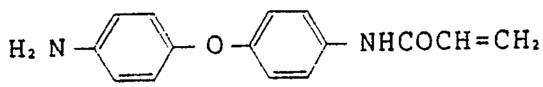

(H-11)

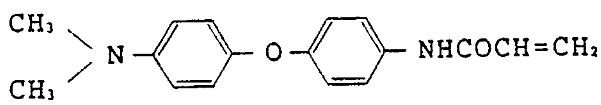

(H-12)

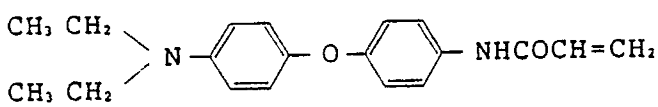

(H-13)

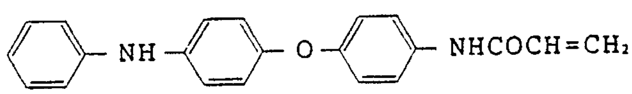

(H-14)

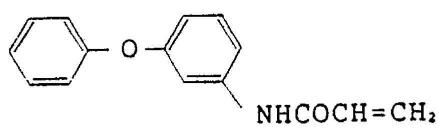

(H-15)

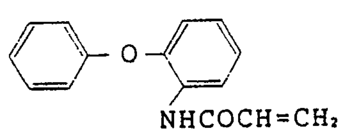

(H-16)

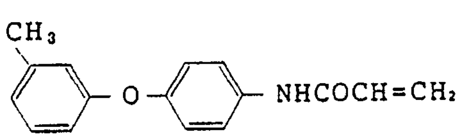

(H-17)

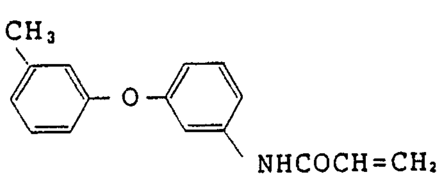

(H-18) 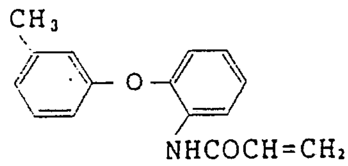

(H-19) 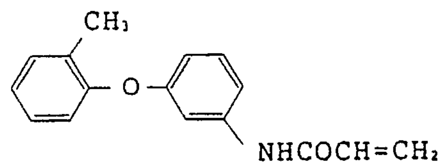

(H-20) 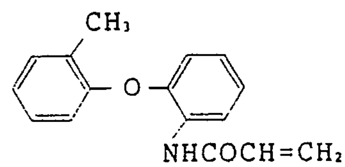

(H-21) 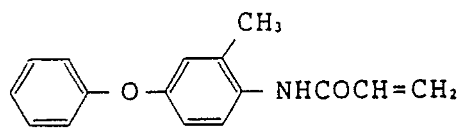

(H-22) 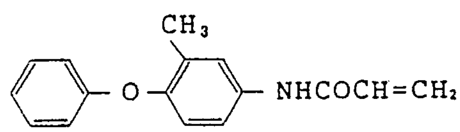

(H-23) 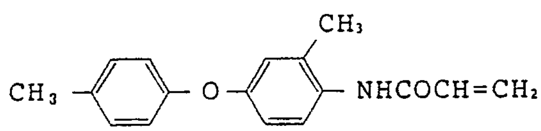

The compound of the above formula (III) can be produced, as described above, by condensation of phenoxyaniline compound represented by formula (IV) with acid halide represented by formula (V) in an inert solvent in the presence of dehydrohalogenating agent.

The starting phenoxyaniline compound represented by the formula (IV) can be prepared by reducing aromatic nitro compound represented by the formula (IVa),

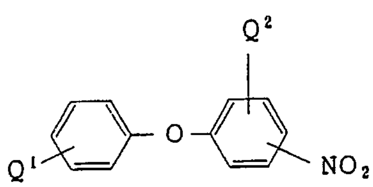

(IVa)

wherein $Q^1$ and $Q^2$ are as defined above. This reduction can be carried out, for example, in an ethanol solvent using a platinum catalyst in accordance with the manner described in J. Am. Chem. Soc., 53 , 1566, 1568 (1931), or in an aqueous hydrochloric acid solution using tin (II) chloride in accordance with the manner described in Monatshefte für Chemie, 57 , 31, 41 (1931).

The aromatic nitro compound represented by the formula (IVa) can be prepared by condensation of phenolic compound represented by the formula (IVb),

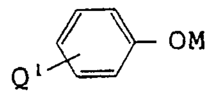

(IVb)

wherein $Q^1$ is as defined above, and M is hydrogen or an alkali metal such as sodium or potassium, but is preferably alkali metal,
with nitrohalobenzene represented by the formula (IVc),

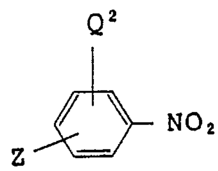

(IVc)

wherein $Q^2$ is as defined above and Z is halogen. This condensation reaction can be carried out, for example, in the presence of a powdery copper catalyst as described in J. Am. Chem. Soc., 55 , 1289 (1933).

The acid halide represented by the above formula (V) can be prepared by reaction of acrylic acid with halogenating agent. Examples of the halogenating agent usable in this reaction are chlorinating agents such as thionyl chloride, phosphorus trichloride and phosphorus pentachloride, brominating agents such as phosphorus tribromide, and the like. The amount of halogenating agent used is normally in a range of from about 1.0 to about 6.0 moles, preferably from about 2.0 to about 5.0 moles, based on 1 mole of the acrylic acid.

The condensation reaction of the phenoxyaniline compound represented by the above formula (IV) with the acid halide represented by the above formula (V) is effected in an inert solvent in the presence of a dehydrohalogenating agent.

The inert solvents usable in this reaction include, for example, aliphatic hydrocarbons such as n-hexane and n-heptane, alicyclic hydrocarbons such as cyclohexane, aromatic hydrocarbons such as benzene, toluene and xylene, esters such as ethyl acetate and butyl acetate, ethers such as diethyl ether, tetrahydrofuran and ethylene glycol dimethyl ether, halogenated hydrocarbons such as dichloromethane, chloroform, carbon tetrachloride and 1,2-dichloroethane, and the like.

Examples of the dehydrohalogenating agent are tertiary amines such as triethylamine, N,N-dimethylaniline, N,N-dimethylbenzylamine and tetramethylurea, pyridine type compounds such as pyridine and 4-(N,N-dimethylamino)pyridine, and the like. The amount of the dehydrohalogenating agent used is preferably in a range of from about 0.8 to about 2.0 moles, more preferably in a range of from about 0.9 to about 1.5 moles, based on 1 mole of the acid halide represented by the foumula (V).

In this reaction, a preferred reaction molar ratio of the acid halide (V) to the phenoxyaniline compound (IV), i.e. (V) : (IV), is normally about 0.7-1.7 : 1, and more preferred is about 0.9-1.3 : 1.

The reaction temperature is normally preferred in a range of from about -40°C to about 100°C, and more preferably in a range of from about -20°C to about 50°C.

After completion of the reaction, an acid salt produced from the dehydrohalogenating agent may be separated by filtration or the like or may not be separated.

The desired product can be separated from the reaction liquor by various methods; for example, the reaction liquor is optionally neutralized and washed with water, and thereafter the solvent is distilled off from the organic layer, or water is added to the reaction liquor and then the liquor is cooled to produce precipitated crystals, or the like. The desired product thus obtained may be further purified by conventional known methods such as recrystallization, washing with solvents, and the like.

When the acrylamide compounds represented by the formula (I) are added to the rubber, they may be used in any form, for example, as a single compound, as a mixture of two or more compounds, as a mixture with carriers including clay which do not affect the rubber properties, and as a mixture with other additives. They can be added at any stage when the compounded rubber is prepared. When the base rubber is a synthetic rubber, they may otherwise be added immediately after polymerization of the synthetic rubber. For example, they can be added to the synthetic rubber after the polymerization in the form of a preliminarily prepared emulsion of the coompound or in the form of a solution which is preliminarily prepared by dissolving the compound in an organic solvent.

On compounding of the rubber, the loading amount of the acrylamide represented by the formula (I) is not limitative, but is generally preferred in a range of from about 0.1 to about 20 parts by weight per 100

parts by weight of the natural and/or synthetic rubber.

The fillers which may used in the present invention include various ones which are used in the rubber industry, but carbon black is normally preferred. The kind of carbon black is not specifically limited, and various kinds of carbon black used heretofore in the rubber industry can be applied. In order to enhance the gripping performance of tires, conventional methods often use a highly reinforcing carbon black having a nitrogen absorption specific surface area of 80 to 250 $m^2$ /g, such as SAF black, ISAF black and HAF black, and such highly reinforcing carbon black is preferably used also in the present invention. The loading amount of the filler is not specifically limited, but is generally preferred in a range of from about 20 to about 200 parts by weight per 100 parts by weight of the rubber.

The rubbers usable in the present invention include, for example, natural rubbers, various synthetic rubbers such as polyisoprene rubber (IR), styrene/butadiene copolymer rubber (SBR), polybutadiene rubber (BR), acrylonitrile/butadiene copolymer rubber (NBR), isoprene/isobutylene copolymer rubber (IIR) and ethylene/propylene/diene copolymer rubber (EPDM), blends of natural rubber with any synthetic rubber, and blends of synthetic rubbers such as SBR with IR and SBR with BR.

In order to enhance the gripping performance of tires, SBR of high styrene content, in particular, has been used recently, and such SBR of high styrene content can also be used in the present invention. The present invention is effective also with SBR having a styrene content of 20 to 50 by weight, and such SBR is one of the preferred rubbers. The SBR may be of solution polymerized or emulsion polymerized type.

On compounding of the rubber material for tire treads, conventional methods often use process oil in order to enhance the gripping performance of tires, and the present invention preferably uses process oil. The loading amount of the process oil is not particularly limited, but is normally not higher than 200 parts by weight per 100 parts by weight of the rubber, and is preferably selected from the range of from 5 to 200 parts by weight. The kinds of process oil are also not limited in the present invention, and various ones used heretofore can be applied.

In the present invention, various rubber chemicals usually used in the rubber industry can, of course, be added, Such rubber chemicals which may be used in the present invention include, for example, antioxidants, vulcanizing agents, vulcanization accelerators, retarders, peptizers, softeners and the like.

According to the present invention, tan $\delta$ at a high temperature of 60 °C or more, which corresponds to gripping power when tires become heated, can be effectively increased without reducing the heat resistance such as blow-out temperature and while maintaining the desired strength characteristics. Therefore, when the rubber composition of the present invention is applied to tires, particularly to tread parts of the tires, the resulting tires can be improved in gripping performance which has close relation to acceleration and braking capability of automobiles.

The present invention is explained below in more detail with reference to Examples in which rubbers are blended with various compounds represented by the above formula (I) to evaluate the rubber properties, but the invention is not limited to these Examples. In the following examples, the percentages and parts are by weight unless otherwise indicated.

Acrylamide compounds used in the Examples are identified as follows :

AA : N-n-Butylacrylamide
AB : N-n-Heptylacrylamide
AC : N-n-Octylacrylamide
AD : N-n-Octadecylacrylamide
AE : N-t-Butylacrylamide
AF : N(1,1,3-Trimethylbutyl)acrylamide
AG : N(1,1,3-Trimethylpentyl)acrylamide
AH : N(1,1,3,3-Tetramethylbutyl)acrylamide
AI : N-Cyclohexylacrylamide
B : N-phenylacrylamide
CA : N-(4-Hydroxyphenyl)acrylamide
CB : N-(4-Methoxyphenyl)acrylamide
CC : N-(4-Ethoxyphenyl)acrylamide
CD : N-(3-Methoxyphenyl)acrylamide
CE : N-(3-Bromo-4-methoxyphenyl)acrylamide
CF : N-(3-cyano-4-methoxyphenyl)acrylamide
DA : N-(4-Chlorophenyl)acrylamide
DB : N-(4-Bromophenyl)acrylamide
DC : N-(3-Chlorophenyl)acrylamide
DD : N-(3-Bromophenyl)acrylamide

DE : N-(3,4-Dibromophenyl)acrylamide
DF : N-(3,5-Dibromophenyl)acrylamide
EA : N-(4-Nitropheny)acrylamide
EB : N-(3-Nitropheny)acrylamide
EC : N-(4-Nitro-3-methylphenyl)acrylamide
ED : N(4-Nitro-3-methoxyphenyl)acrylamide
EE : N-(4-Nitro-3-bromophenyl)acrylamide
EF : N-(3,4-Dinitrophenyl)acrylamide
FA : N-(4-Methylphenyl)acrylamide
FB : N-(4-Ethylphenyl)acrylamide
FC : N-(4-Octylphenyl)acrylamide
FD : N-(3-Methylphenyl)acrylamide
FE : N-(3-Methoxy-4-methylphenyl)acrylamide
FF : N-(3-Hydroxy-4-t-butylphenyl)acrylamide
GA : N-(4-Cyanophenyl)acrylamide
GB : N-(4-Methoxycarbonylphenyl)acrylamide
GC : N-(4-Ethoxycarbonylphenyl)acrylamide
GD : N-(3-Cyanophenyl)acrylamide
GE : N-(2-Cyanophenyl)acrylamide
GF : N-(3-Ethoxycarbonylphenyl)acrylamide
HA : 4-phenoxyphenylacrylamide
HB : 4-(4-Methylphenoxy)phenylacrylamide
HC : 4-(4-Chlorophenoxy)phenylacrylamide
HD : 3-phenoxyphenylacrylamide
HE : 4-(4-N,N-Dimethylaminophenoxy)phenylacrylamide

Example 1

| [Compounding formulation] | |
|---|---|
| Styrene/butadiene copolymer rubber (styrene content of 25 %) | 100 parts |
| ISAF carbon black | 50 parts |
| Stearic acid | 3 parts |
| Zinc oxide | 5 parts |
| Aromatic process oil | shown in Table 1 |
| Antioxidant (N-Phenyl-N′-1,3-dimethylbutyl-p-phenylenediamine) | 1 part |
| Vulcanization accelerator (N-Cyclohexyl-2-benzothiazylsulfenamide) | 1 part |
| Sulfur | 2 parts |
| Acrylamide (AA through AI) | shown in Table 1 |

Using 250 ml Laboplastomill® manufactured by Toyo Seiki Co. as a Bumbury's mixer, and at an oil bath temperature of 170° C, thereto was charged the basal styrene/butadiene copolymer rubber, the acrylamide of the present invention, carbon black, stearic acid, process oil, antioxidant and zinc oxide in accordance with the above compounding formulation, and the mixture was kneaded for 5 minutes with a mixer revolution of 60 rpm. The rubber temperature at the kneading was 160° - 170° C.

The rubber blend was then transferred to an open mill, and thereto was added the vulcanization accelerator and sulfur shown in the above formulation at a temperature of 40° - 50° C, followed by kneading.

The kneaded mixture was then vulcanized at 150° C for 50 minutes using a vulcanizing press to form a predetermined shape, and the shaped vulcanized composition was subjected to the measurement of tan $\delta$. Tan $\delta$ was determined using a viscoelasticity spectrometer manufactured by Iwamoto seisakusho Co. under a frequency of 10 Hz and at respective temperatures of from a room temperature to 100° C.

A heat resistance test was also conducted by measuring a blow-out temperature for a vulcanized rubber specimen of about 1 cm x 1 cm x 3.5 mm which was prepared from the above rubber composition. Thus, the vulcanized rubber specimen was put in a thermostat, and the blow-out temperature was determined by

such a method that while elevating the temperature from 200 °C to 300 °C at regular intervals of 10 °C, the specimen was left at each temperature for 1 hour, thereafter was taken out and cut in half to visually observe whether bubbles were grown inside the specimen. A temperature at which the bubbles were observed for the first time was determined as the blow-out temperature.

Of the test results obtained, tan $\delta$ at 80 °C and the blow-out temperature were shown in Table 1 below together with the compounding condition of the acrylamide and process oil.

Table 1

| | Run No. | Invention | | | | | |
|---|---|---|---|---|---|---|---|
| | | 1 | 2 | 3 | 4 | 5 | 6 |
| Compounding | Acrylamide | AA | AB | AC | AD | AE | AF |
| | Amount of acrylamide (parts) | 5 | 5 | 5 | 5 | 5 | 5 |
| | Amount of aromatic process oil (parts) | – | – | – | – | – | – |
| Result | Tan $\delta$ at 80°C | 0.165 | 0.167 | 0.169 | 0.167 | 0.192 | 0.193 |
| | Blow-out temperature (°C) | 260 | 270 | 270 | 270 | 280 | 280 |

(to be cont'd)

Table 1 (cont'd)

| Invention | | | | | | Comparison | | |
|---|---|---|---|---|---|---|---|---|
| 7 | 8 | 9 | 10 | 11 | 12 | 13 | 14 | 15 |
| AG | AH | AH | AH | AH | AI | — | — | — |
| 5 | 5 | 10 | 5 | 5 | 5 | — | — | — |
| — | — | — | 10 | 20 | — | — | 10 | 20 |
| 0.198 | 0.199 | 0.203 | 0.203 | 0.206 | 0.174 | 0.141 | 0.149 | 0.159 |
| 280 | 280 | 280 | 270 | 270 | 260 | 280 | 280 | 270 |

Example 2

| [Compounding formulation] | |
|---|---|
| Styrene/butadiene copolymer rubber (having a styrene content of 35 % and containing 37.5 parts of aromatic oil per 100 parts of the rubber) | shown in Table 2 |
| Natural rubber | shown in Table 2 |
| Butadiene rubber (BR-01) | shown in Table 2 |
| SAF carbon black | 65 parts |
| Stearic acid | 1 part |
| Zinc oxide | 3 parts |
| Aromatic process oil | 40 parts |
| Antioxidant (the same as that in Example 1) | 1 part |
| Vulcanization accelerator (the same as that in Example 1) | 1 part |
| Sulfur | 2 parts |
| Acrylamide | shown in Table 2 |

Vulcanized rubber was prepared based on the above compounding formulation and in concordance with the manner in Example 1, and it was subjected to the same tests as in Example 1. The results obtained were shown in Table 2 below together with the compounding condition of the base rubber and acrylamide.

Table 2

| | Run No. | Invention | | | | | | | Comparison | | |
|---|---|---|---|---|---|---|---|---|---|---|---|
| | | 1 | 2 | 3 | 4 | 5 | 6 | 7 | 8 | 9 | 10 |
| Compounding | Styrene/butadiene copolymer rubber (parts) | 137.5 | 137.5 | 137.5 | 137.5 | 137.5 | – | – | 137.5 | – | – |
| | Natural rubber (parts) | – | – | – | – | – | 100 | 70 | – | 100 | 70 |
| | Butadiene rubber (parts) | – | – | – | – | – | – | 30 | – | – | 30 |
| | Acrylamide | AA | AC | AE | AG | AH | AH | AH | – | – | – |
| | Amount of acrylamide (parts) | 5 | 5 | 5 | 5 | 5 | 5 | 5 | – | – | – |
| Result | Tan $\delta$ at 80°C | 0.197 | 0.201 | 0.228 | 0.232 | 0.239 | 0.175 | 0.178 | 0.183 | 0.142 | 0.147 |
| | Blow-out temperature (°C) | 250 | 250 | 260 | 270 | 270 | 190 | 220 | 270 | 190 | 220 |

Example 3

| [Compounding formulation] | |
|---|---|
| Styrene/butadiene copolymer rubber (styrene content of 25 %) | 100 parts |
| ISAF carbon black | 50 parts |
| Stearic acid | 3 parts |
| Zinc oxide | 5 parts |
| Aromatic process oil | shown in Table 3 |
| Antioxidant (N-Phenyl-N′-1,3-dimethylbutyl-p-phenylenediamine) | 1 part |
| Vulcanization accelerator (N-Cyclohexyl-2-benzothiazylsulfenamide) | 1 part |
| Sulfur | 2 parts |
| Acrylamide (B) | shown in Table 3 |

Using 250 ml Laboplastomill® manufactured by Toyo Seiki Co. as a Bumbury's mixer, and at an oil bath temperature of 170° C, thereto was charged the basal styrene/butadiene copolymer rubber, subjective acrylamide of the present invention, carbon black, stearic acid, process oil, antioxidant and zinc oxide in accordance with the above compounding formulation, and the mixture was kneaded for 5 minutes with a mixer revolution of 60 rpm. The rubber temperature at the kneading was 160° - 170° C.

The rubber blend was then transferred to an open mill, and thereto was added the vulcanization accelerator and sulfur shown in the above formulation at a temperature of 40° - 50° C, followed by kneading.

The kneaded mixture was then vulcanized at 150° C for 50 minutes using a vulcanizing press to form a predetermined shape, and the shaped vulcanized composition was subjected to the measurement of tan $\delta$ . Tan $\delta$ was determined using a viscoelasticity spectrometer manufactured by Iwamoto Seisakusho Co. under a frequency of 10 Hz and at respective temperatures of from a room temperature to 100° C.

A heat resistance test was also conducted by measuring a blow-out temperature for a vulcanized rubber specimen of about 1 cm x 1 cm x 3.5 mm which was prepared from the above rubber composition. Thus, the vulcanized rubber specimen was put in a thermostat, and the blow-out temperature was determined by such a method that while elevating the temperature from 200° C to 300° C at regular intervals of 10° C, the specimen was left at each temperature for 1 hour, thereafter was taken out and cut in half to visually observe whether bubbles were grown inside the specimen. A temperature at which the bubbles were observed for the first time was determined as the blow-out temperature.

Of the test results obtained, tan $\delta$ at 80° C and the blow-out temperature were shown in Table 3 below together with the compounding condition of the acrylamide and process oil.

Table 3

| | Run No. | Invention | | | | Comparison | | |
|---|---|---|---|---|---|---|---|---|
| | | 1 | 2 | 3 | 4 | 5 | 6 | 7 |
| Compound-ing | Acrylamide | B | B | B | B | — | — | — |
| | Amount of acrylamide (parts) | 5 | 10 | 5 | 5 | — | — | — |
| | Amount of aromatic process oil (parts) | — | — | 10 | 20 | — | 10 | 20 |
| Result | Tan $\delta$ at 80°C | 0.200 | 0.208 | 0.204 | 0.213 | 0.138 | 0.147 | 0.155 |
| | Blow-out temperature (°C) | 280 | 280 | 270 | 270 | 280 | 270 | 270 |

Example 4

| [Compounding formulation] | |
|---|---|
| Styrene/butadiene copolymer rubber (having a styrene content of 35 % and containing 37.5 parts of aromatic oil per 100 parts of the rubber) | shown in Table 4 |
| Natural rubber | shown in Table 4 |
| Butadiene rubber (BR-01) | shown in Table 4 |
| SAF carbon black | 65 parts |
| Stearic acid | 1 part |
| Zinc oxide | 3 parts |
| Aromatic process oil | 40 parts |
| Antioxidant (the same as that in Example 3) | 1 part |
| Vulcanization accelerator (the same as that in Example 3) | 1 part |
| Sulfur | 2 parts |
| Acrylamide (B) | shown in Table 4 |

Vulcanized rubber was prepared based on the above compounding formulation and in concordance with the manner in Example 3, and it was subjected to the same tests as in Example 3. The results obtained were shown in Table 4 below together with the compounding condition of the base rubber and acrylamide.

Table 4

| | Run No. | Invention 1 | Invention 2 | Invention 3 | Invention 4 | Comparison 5 | Comparison 6 | Comparison 7 |
|---|---|---|---|---|---|---|---|---|
| Compounding | Styrene/butadiene copolymer rubber (parts) | 137.5 | 137.5 | - | - | 137.5 | - | - |
| | Natural rubber (parts) | - | - | 100 | 70 | - | 100 | 70 |
| | Butadiene rubber (parts) | - | - | - | 30 | - | - | 30 |
| | Acrylamide | B | B | B | B | - | - | - |
| | Amount of acrylamide (parts) | 5 | 10 | 5 | 5 | - | - | - |
| Result | Tan δ at 80°C | 0.244 | 0.261 | 0.171 | 0.176 | 0.181 | 0.144 | 0.149 |
| | Blow-out temperature (°C) | 270 | 260 | 190 | 220 | 270 | 190 | 220 |

Example 5

| [Compounding formulation] | |
|---|---|
| Styrene/butadiene copolymer rubber (styrene content of 25 %) | 100 parts |
| ISAF carbon black | 50 parts |
| Stearic acid | 3 parts |
| Zinc oxide | 5 parts |
| Aromatic process oil | shown in Table 5 |
| Antioxidant (N-Phenyl-N′-1,3-dimethylbutyl-p-phenylenediamine) | 1 part |
| Vulcanization accelerator (N-Cyclohexyl-2-benzothiazylsulfenamide) | 1 part |
| Sulfur | 2 parts |
| Acrylamide (CA through CF) | shown in Table 5 |

Using 250 ml Laboplastomill® manufactured by Toyo Seiki Co. as a Bumbury's mixer, and at an oil bath temperature of 170° C, thereto was charged the basal styrene/butadiene copolymer rubber, subjective acrylamide of the present invention, carbon black, stearic acid, process oil, antioxidant and zinc oxide in accordance with the above compounding formulation, and the mixture was kneaded for 5 minutes with a mixer revolution of 60 rpm. The rubber temperature at the kneading was 160° - 170° C.

The rubber blend was then transferred to an open mill, and thereto was added the vulcanization accelerator and sulfur shown in the above formulation at a temperature of 40° - 50° C, followed by kneading.

The kneaded mixture was then vulcanized at 150° C for 50 minutes using a vulcanizing press to form a predetermined shape, and the shaped vulcanized composition was subjected to the measurement of tan $\delta$. Tan $\delta$ was determined using a viscoelasticity spectrometer manufactured by Iwamoto Seisakusho Co. under a frequency of 10 Hz and at respective temperatures of from a room temperature to 100° C.

A heat resistance test was also conducted by measuring a blow-out temperature for a vulcanized rubber specimen of about 1 cm x 1 cm x 3.5 mm which was prepared from the above rubber composition. Thus, the vulcanized rubber specimen was put in a thermostat, and the blow-out temperature was determined by such a method that while elevating the temperature from 200° C to 300° C at regular intervals of 10° C, the specimen was left at each temperature for 1 hour, thereafter was taken out and cut in half to visually observe whether bubbles were grown inside the specimen. A temperature at which the bubbles were observed for the first time was determined as the blow-out temperature.

Of the test results obtained, tan $\delta$ at 80° C and the blow-out temperature were shown in Table 5 below together with the compounding condition of the acrylamide and process oil.

Table 5

| Run No. | | Invention | | | | | |
|---|---|---|---|---|---|---|---|
| | | 1 | 2 | 3 | 4 | 5 | 6 |
| Compounding | Acrylamide | CA | CA | CB | CB | CB | CB |
| | Amount of acrylamide (parts) | 5 | 5 | 5 | 10 | 5 | 5 |
| | Amount of aromatic process oil (parts) | — | 20 | — | — | 10 | 20 |
| Result | Tan $\delta$ at 80°C | 0.198 | 0.204 | 0.200 | 0.203 | 0.202 | 0.205 |
| | Blow-out temperature (°C) | 270 | 270 | 280 | 280 | 270 | 270 |

(to be cont'd)

Table 5 (cont'd)

| | | | | | | | | | |
|---|---|---|---|---|---|---|---|---|---|
| | Invention | | | | | | Comparison | | |
| | 7 | 8 | 9 | 10 | 11 | 12 | 13 | 14 | 15 |
| | CC | CC | CC | CD | CE | CF | — | — | — |
| | 5 | 10 | 5 | 5 | 5 | 5 | — | — | — |
| | — | — | 20 | — | — | 20 | — | 10 | 20 |
| | 0.201 | 0.204 | 0.204 | 0.175 | 0.184 | 0.175 | 0.138 | 0.147 | 0.155 |
| | 280 | 280 | 270 | 270 | 260 | 270 | 280 | 270 | 270 |

Example 6

| [Compounding formulation] | |
|---|---|
| Styrene/butadiene copolymer rubber (having a styrene content of 35 % and containing 37.5 parts of aromatic oil per 100 parts of the rubber) | shown in Table 6 |
| Natural rubber | shown in Table 6 |
| Butadiene rubber (BR-01) | shown in Table 6 |
| SAF carbon black | 65 parts |
| Stearic acid | 1 part |
| Zinc oxide | 3 parts |
| Aromatic process oil | 40 parts |
| Antioxidant (the same as that in Example 5) | 1 part |
| Vulcanization accelerator (the same as that in Example 5) | 1 part |
| Sulfur | 2 parts |
| Acrylamide | shown in Table 6 |

Vulcanized rubber was prepared based on the above compounding formulation and in concordance with the manner in Example 5, and it was subjected to the same tests as in Example 5. The results obtained were shown in Table 6 below together with the compounding condition of the base rubber and acrylamide.

Table 6

| | Run No. | Invention | | | | | | Comparison | | |
|---|---|---|---|---|---|---|---|---|---|---|
| | | 1 | 2 | 3 | 4 | 5 | 6 | 7 | 8 | 9 |
| Compounding | Styrene/ butadiene copolymer rubber (parts) | 137.5 | 137.5 | 137.5 | 137.5 | — | — | 137.5 | — | — |
| | Natural rubber (parts) | — | — | — | — | 100 | 70 | — | 100 | 70 |
| | Butadiene rubber (parts) | — | — | — | — | — | 30 | — | — | 30 |
| | Acrylamide | CA | CB | CC | CD | CB | CB | — | — | — |
| | Amount of acrylamide (parts) | 5 | 5 | 5 | 5 | 5 | 5 | — | — | — |
| Result | Tan $\delta$ at 80°C | 0.235 | 0.243 | 0.241 | 0.193 | 0.174 | 0.180 | 0.181 | 0.144 | 0.149 |
| | Blow-out temperature (°C) | 270 | 270 | 270 | 250 | 180 | 210 | 270 | 190 | 220 |

Example 7

| [Compounding formulation] | |
|---|---|
| Styrene/butadiene copolymer rubber (styrene content of 25 %) | 100 parts |
| ISAF carbon black | 50 parts |
| Stearic acid | 3 parts |
| Zinc oxide | 5 parts |
| Aromatic process oil | shown in Table 7 |
| Antioxidant (N-Phenyl-N′-1,3-dimethylbutyl-p-phenylenediamine) | 1 part |
| Vulcanization accelerator (N-Cyclohexyl-2-benzothiazylsulfenamide) | 1 part |
| Sulfur | 2 parts |
| Acrylamide (DA through DF) | shown in Table 7 |

Using 250 ml Laboplastomill® manufactured by Toyo Seiki Co. as a Bumbury's mixer, and at an oil bath temperature of 170° C, thereto was charged the basal styrene/butadiene copolymer rubber, subjective acrylamide of the present invention, carbon black, stearic acid, process oil, antioxidant and zinc oxide in accordance with the above compounding formulation, and the mixture was kneaded for 5 minutes with a mixer revolution of 60 rpm. The rubber temperature at the kneading was 160° - 170° C.

The rubber blend was then transferred to an open mill, and thereto was added the vulcanization accelerator and sulfur shown in the above formulation at a temperature of 40° - 50° C, followed by kneading.

The kneaded mixture was then vulcanized at 150° C for 50 minutes using a vulcanizing press to form a predetermined shape, and the shaped vulcanized composition was subjected to the measurement of tan $\delta$. Tan $\delta$ was determined using a viscoelasticity spectrometer manufactured by Iwamoto Seisakusho Co. under a frequency of 10 Hz and at respective temperatures of from a room temperature to 100° C.

A heat resistance test was also conducted by measuring a blow-out temperature for a vulcanized rubber specimen of about 1 cm x 1 cm x 3.5 mm which was prepared from the above rubber composition. Thus, the vulcanized rubber specimen was put in a thermostat, and the blow-out temperature was determined by such a method that while elevating the temperature from 200° C to 300° C at regular intervals of 10° C, the specimen was left at each temperature for 1 hour, thereafter was taken out and cut in half to visually observe whether bubbles were grown inside the specimen. A temperature at which the bubbles were observed for the first time was determined as the blow-out temperature.

Of the test results obtained, tan $\delta$ at 80° C and the blow-out temperature were shown in Table 7 below together with the compounding condition of the acrylamide and process oil.

Table 7

| | Run No. | Invention | | | | | |
|---|---|---|---|---|---|---|---|
| | | 1 | 2 | 3 | 4 | 5 | 6 |
| Compounding | Acrylamide | DA | DA | DA | DA | DB | DB |
| | Amount of acrylamide (parts) | 5 | 10 | 5 | 5 | 5 | 5 |
| | Amount of aromatic process oil (parts) | — | — | 10 | 20 | — | 20 |
| Result | Tan $\delta$ at 80°C | 0.200 | 0.207 | 0.206 | 0.210 | 0.201 | 0.212 |
| | Blow-out temperature (°C) | 280 | 270 | 270 | 270 | 280 | 270 |

(to be cont'd)

Table 7 (cont'd)

| Invention | | | | | | Comparison | | |
|---|---|---|---|---|---|---|---|---|
| 7 | 8 | 9 | 10 | 11 | 12 | 13 | 14 | 15 |
| DC | DC | DD | DD | DE | DF | — | — | — |
| 5 | 5 | 5 | 5 | 5 | 5 | — | — | — |
| — | 20 | — | 20 | — | — | — | 10 | 20 |
| 0.198 | 0.211 | 0.194 | 0.197 | 0.183 | 0.181 | 0.138 | 0.147 | 0.155 |
| 270 | 270 | 270 | 260 | 270 | 270 | 280 | 270 | 270 |

Example 8

| [Compounding formulation] | |
|---|---|
| Styrene/butadiene copolymer rubber (having a styrene content of 35 % and containing 37.5 parts of aromatic oil per 100 parts of the rubber) | shown in Table 8 |
| Natural rubber | shown in Table 8 |
| Butadiene rubber (BR-01) | shown in Table 8 |
| SAF carbon black | 65 parts |
| Stearic acid | 1 part |
| Zinc oxide | 3 parts |
| Aromatic process oil | 40 parts |
| Antioxidant (the same as that in Example 7) | 1 part |
| Vulcanization accelerator (the same as that in Example 7) | 1 part |
| Sulfur | 2 parts |
| Acrylamide | shown in Table 8 |

Vulcanized rubber was prepared based on the above compounding formulation and in concordance with the manner in Example 7, and it was subjected to the same tests as in Example 7. The results obtained were shown in Table 8 below together with the compounding condition of the base rubber and acrylamide.

Table 8

| | Run No. | Invention | | | | | | Comparison | | |
|---|---|---|---|---|---|---|---|---|---|---|
| | | 1 | 2 | 3 | 4 | 5 | 6 | 7 | 8 | 9 |
| Compound-ing | Styrene/ butadiene copolymer rubber (parts) | 137.5 | 137.5 | 137.5 | 137.5 | — | — | 137.5 | — | — |
| | Natural rubber (parts) | — | — | — | — | 100 | 70 | — | 100 | 70 |
| | Butadiene rubber (parts) | — | — | — | — | — | 30 | — | — | 30 |
| | Acrylamide | DA | DB | DC | DD | DA | DA | — | — | — |
| | Amount of acrylamide (parts) | 5 | 5 | 5 | 5 | 5 | 5 | — | — | — |
| Result | Tan $\delta$ at 80°C | 0.243 | 0.244 | 0.236 | 0.235 | 0.177 | 0.181 | 0.181 | 0.144 | 0.149 |
| | Blow-out temperature (°C) | 270 | 270 | 270 | 270 | 190 | 220 | 270 | 190 | 220 |

Example 9

| [Compounding formulation] | |
|---|---|
| Styrene/butadiene copolymer rubber (styrene content of 25 %) | 100 parts |
| ISAF carbon black | 50 parts |
| Stearic acid | 3 parts |
| Zinc oxide | 5 parts |
| Aromatic process oil | shown in Table 9 |
| Antioxidant (N-Phenyl-N′-1,3-dimethylbutyl-p-phenylenediamine) | 1 part |
| Vulcanization accelerator (N-Cyclohexyl-2-benzothiazylsulfenamide) | 1 part |
| Sulfur | 2 parts |
| Acrylamide (EA through EF) | shown in Table 9 |

Using 250 ml Laboplastomill® manufactured by Toyo Seiki Co. as a Bumburyl's mixer, and at an oil bath temperature of 170° C, thereto was charged the basal styrene/butadiene copolymer rubber, subjective acrylamide of the present invention, carbon black, stearic acid, process oil, antioxidant and zinc oxide in accordance with the above compounding formulation, and the mixture was kneaded for 5 minutes with a mixer revolution of 60 rpm. The rubber temperature at the kneading was 160° - 170° C.

The rubber blend was then transferred to an open mill, and thereto was added the vulcanization accelerator and sulfur shown in the above formulation at a temperature of 40° - 50° C, followed by kneading.

The kneaded mixture was then vulcanized at 150° C for 50 minutes using a vulcanizing press to form a predetermined shape, and the shaped vulcanized composition was subjected to the measurement of tan $\delta$. Tan $\delta$ was determined using a viscoelasticity spectrometer manufactured by Iwamoto Seisakusho Co. under a frequency of 10 Hz and at respective temperatures of from a room temperature to 100° C.

A heat resistance test was also conducted by measuring a blow-out temperature for a vulcanized rubber specimen of about 1 cm x 1 cm x 3.5 mm which was prepared from the above rubber composition. Thus, the vulcanized rubber specimen was put in a thermostat, and the blow-out temperature was determined by such a method that while elevating the temperature from 200° C to 300° C at regular intervals of 10° C, the specimen was left at each temperature for 1 hour, thereafter was taken out and cut in half to visually observe whether bubbles were grown inside the specimen. A temperature at which the bubbles were observed for the first time was determined as the blow-out temperature.

Of the test results obtained, tan $\delta$ at 80° C and the blow-out temperature were shown in Table 9 below together with the compounding condition of the acrylamide and process oil.

Table 9

| | Run No. | Invention | | | | | |
|---|---|---|---|---|---|---|---|
| | | 1 | 2 | 3 | 4 | 5 | 6 |
| Compounding | Acrylamide | EA | EA | EA | EA | EB | EB |
| | Amount of acrylamide (parts) | 5 | 10 | 5 | 5 | 5 | 5 |
| | Amount of aromatic process oil (parts) | — | — | 10 | 20 | — | 20 |
| Result | Tan $\delta$ at 80°C | 0.195 | 0.205 | 0.201 | 0.207 | 0.196 | 0.206 |
| | Blow-out temperature (°C) | 280 | 280 | 270 | 270 | 280 | 270 |

(to be cont'd)

Table 9 (cont'd)

| Invention | | | | | | Comparison | | |
|---|---|---|---|---|---|---|---|---|
| 7 | 8 | 9 | 10 | 11 | 12 | 13 | 14 | 15 |
| EC | ED | ED | EE | EF | EF | — | — | — |
| 5 | 5 | 5 | 5 | 5 | 5 | — | — | — |
| — | — | 20 | — | — | 20 | — | 10 | 20 |
| 0.178 | 0.191 | 0.203 | 0.181 | 0.192 | 0.202 | 0.138 | 0.147 | 0.155 |
| 270 | 260 | 280 | 270 | 270 | 260 | 280 | 270 | 270 |

Example 10

| [Compounding formulation] | |
|---|---|
| Styrene/butadiene copolymer rubber (having a styrene content of 35 % and containing 37.5 parts of aromatic oil per 100 parts of the rubber) | shown in Table 10 |
| Natural rubber | shown in Table 10 |
| Butadiene rubber (BR-01) | shown in Table 10 |
| SAF carbon black | 65 parts |
| Stearic acid | 1 part |
| Zinc oxide | 3 parts |
| Aromatic process oil | 40 parts |
| Antioxidant (the same as that in Example 9) | 1 part |
| Vulcanization accelerator (the same as that in Example 9) | 1 part |
| Sulfur | 2 parts |
| Acrylamide | shown in Table 10 |

Vulcanized rubber was prepared based on the above compounding formulation and in concordance with the manner in Example 9, and it was subjected to the same tests as in Example 9. The results obtained were shown in Table 10 below together with the compounding condition of the base rubber and acrylamide.

Table 10

| | Run No. | Invention | | | | | | Comparison | | |
|---|---|---|---|---|---|---|---|---|---|---|
| | | 1 | 2 | 3 | 4 | 5 | 6 | 7 | 8 | 9 |
| Compounding | Styrene/butadiene copolymer rubber (parts) | 137.5 | 137.5 | 137.5 | — | — | — | 137.5 | — | — |
| | Natural rubber (parts) | — | — | — | 100 | 70 | 70 | — | 100 | 70 |
| | Butadiene rubber (parts) | — | — | — | — | 30 | 30 | — | — | 30 |
| | Acrylamide | EA | EA | EB | EA | EA | EB | — | — | — |
| | Amount of acrylamide (parts) | 5 | 10 | 5 | 5 | 5 | 5 | — | — | — |
| Result | Tan $\delta$ at 80°C | 0.234 | 0.241 | 0.236 | 0.173 | 0.179 | 0.181 | 0.181 | 0.144 | 0.149 |
| | Blow-out temperature (°C) | 270 | 260 | 270 | 190 | 210 | 210 | 270 | 190 | 220 |

Example 11

| [Compounding formulation] | |
|---|---|
| Styrene/butadiene copolymer rubber (styrene content of 25 %) | 100 parts |
| ISAF carbon black | 50 parts |
| Stearic acid | 3 parts |
| Zinc oxide | 5 parts |
| Aromatic process oil | shown in Table 11 |
| Antioxidant (N-Phenyl-N′-1,3-dimethylbutyl-p-phenylenediamine) | 1 part |
| Vulcanization accelerator (N-Cyclohexyl-2-benzothiazylsulfenamide) | 1 part |
| Sulfur | 2 parts |
| Acrylamide (FA through FF) | shown in Table 11 |

Using 250 ml Laboplastomill® manufactured by Toyo Seiki Co. as a Bumbury's mixer, and at an oil bath temperature of 170°C, thereto was charged the basal styrene/butadiene copolymer rubber, subjective acrylamide of the present invention, carbon black, stearic acid, process oil, antioxidant and zinc oxide in accordance with the above compounding formulation, and the mixture was kneaded for 5 minutes with a mixer revolution of 60 rpm. The rubber temperature at the kneading was 160° - 170°C.

The rubber blend was then transferred to an open mill, and thereto was added the vulcanization accelerator and sulfur shown in the above formulation at a temperature of 40° - 50°C, followed by kneading.

The kneaded mixture was then vulcanized at 150°C for 50 minutes using a vulcanizing press to form a predetermined shape, and the shaped vulcanized composition was subjected to the measurement of tan $\delta$. Tan $\delta$ was determined using a viscoelasticity spectrometer manufactured by Iwamoto seisakusho Co. under a frequency of 10 Hz and at respective temperatures of from a room temperature to 100°C.

A heat resistance test was also conducted by measuring a blow-out temperature for a vulcanized rubber specimen of about 1 cm x 1 cm x 3.5 mm which was prepared from the above rubber composition. Thus, the vulcanized rubber specimen was put in a thermostat, and the blow-out temperature was determined by such a method that while elevating the temperature from 200°C to 300°C at regular intervals of 10°C, the specimen was left at each temperature for 1 hour, thereafter was taken out and cut in half to visually observe whether bubbles were grown inside the specimen. A temperature at which the bubbles were observed for the first time was determined as the blow-out temperature.

Of the test results obtained, tan $\delta$ at 80°C and the blow-out temperature were shown in Table 11 below together with the compounding condition of the acrylamide and process oil.

Table 11

| Run No. | | Invention | | | | | |
|---|---|---|---|---|---|---|---|
| | | 1 | 2 | 3 | 4 | 5 | 6 |
| Compounding | Acrylamide | FA | FA | FA | FA | FB | FB |
| | Amount of acrylamide (parts) | 5 | 10 | 5 | 5 | 5 | 5 |
| | Amount of aromatic process oil (parts) | – | – | 10 | 20 | – | 20 |
| Result | Tan $\delta$ at 80°C | 0.193 | 0.201 | 0.201 | 0.203 | 0.191 | 0.199 |
| | Blow-out temperature (°C) | 280 | 280 | 270 | 270 | 280 | 270 |

(to be cont'd)

Table 11 (cont'd)

| Invention | | | | | | Comparison | | |
|---|---|---|---|---|---|---|---|---|
| 7 | 8 | 9 | 10 | 11 | 12 | 13 | 14 | 15 |
| FC | FD | FD | FE | FF | FF | – | – | – |
| 5 | 5 | 5 | 5 | 5 | 5 | – | – | – |
| – | – | 20 | – | – | 20 | – | 10 | 20 |
| 0.171 | 0.175 | 0.181 | 0.165 | 0.173 | 0.180 | 0.138 | 0.147 | 0.155 |
| 260 | 270 | 260 | 280 | 280 | 280 | 280 | 270 | 270 |

Example 12

| [Compounding formulation] | |
|---|---|
| Styrene/butadiene copolymer rubber (having a styrene content of 35 % and containing 37.5 parts of aromatic oil per 100 parts of the rubber) | shown in Table 12 |
| Natural rubber | shown in Table 12 |
| Butadiene rubber (BR-01) | shown in Table 12 |
| SAF carbon black | 65 parts |
| Stearic acid | 1 part |
| Zinc oxide | 3 parts |
| Aromatic process oil | 40 parts |
| Antioxidant (the same as that in Example 11) | 1 part |
| Vulcanization accelerator (the same as that in Example 11) | 1 part |
| Sulfur | 2 parts |
| Acrylamide | shown in Table 12 |

Vulcanized rubber was prepared based on the above compounding formulation and in concordance with the manner in Example 11, and it was subjected to the same tests as in Example 11. The results obtained were shown in Table 12 below together with the compounding condition of the base rubber and acrylamide.

Table 12

| | Run No. | Invention | | | | | | Comparison | | |
|---|---|---|---|---|---|---|---|---|---|---|
| | | 1 | 2 | 3 | 4 | 5 | 6 | 7 | 8 | 9 |
| Compounding | Styrene/butadiene copolymer rubber (parts) | 137.5 | 137.5 | 137.5 | 137.5 | – | – | 137.5 | – | – |
| | Natural rubber (parts) | – | – | – | – | 100 | 70 | – | 100 | 70 |
| | Butadiene rubber (parts) | – | – | – | – | – | 30 | – | – | 30 |
| | Acrylamide | FA | FA | FB | FC | FA | FA | – | – | – |
| | Amount of acrylamide (parts) | 5 | 10 | 5 | 5 | 5 | 5 | – | – | – |
| Result | Tan δ at 80°C | 0.232 | 0.243 | 0.230 | 0.218 | 0.174 | 0.179 | 0.181 | 0.144 | 0.149 |
| | Blow-out temperature (°C) | 270 | 260 | 270 | 270 | 190 | 210 | 270 | 190 | 220 |

Example 13

| [Compounding formulation] | |
|---|---|
| Styrene/butadiene copolymer rubber (styrene content of 25 %) | 100 parts |
| ISAF carbon black | 50 parts |
| Stearic acid | 3 parts |
| Zinc oxide | 5 parts |
| Aromatic process oil | shown in Table 13 |
| Antioxidant (N-Phenyl-N′-1,3-dimethylbutyl-p-phenylenediamine) | 1 part |
| Vulcanization accelerator (N-Cyclohexyl-2-benzothiazylsulfenamide) | 1 part |
| Sulfur | 2 parts |
| Acrylamide (GA through GF) | shown in Table 13 |

Using 250 ml Laboplastomill® manufactured by Toyo Seiki Co. as a Bumbury's mixer, and at an oil bath temperature of 170° C, thereto was charged the basal styrene/butadiene copolymer rubber, subjective acrylamide of the present invention, carbon black, stearic acid, process oil, antioxidant and zinc oxide in accordance with the above compounding formulation, and the mixture was kneaded for 5 mimutes with a mixer revolution of 60 rpm. The rubber temperature at the kneading was 160° - 170° C.

The rubber blend was then transferred to an open mill, and thereto was added the vulcanization accelerator and sulfur shown in the above formulation at a temperature of 40° - 50° C, followed by kneading.

The kneaded mixture was then vulcanized at 150° C for 50 minutes using a vulcanizing press to form a predetermined shape, and the shaped vulcanized composition was subjected to the measurement of tan $\delta$. Tan $\delta$ was determined using a viscoelasticity spectrometer manufactured by Iwamoto Seisakusho Co. under a frequency of 10 Hz and at respective temperatures of from a room temperature to 100° C.

A heat resistance test was also conducted by measuring a blow-out temperature for a vulcanized rubber specimen of about 1 cm x 1 cm x 3.5 mm which was prepared from the above rubber composition. Thus, the vulcanized rubber specimen was put in a thermostat, and the blow-out temperature was determined by such a method that while elevating the temperature from 200° C to 300° C at regular intervals of 10° C, the specimen was left at each temperature for 1 hour, thereafter was taken out and cut in half to visually observe whether bubbles were grown inside the specimen. A temperature at which the bubbles were observed for the first time was determined as the blow-out temperature.

Of the test results obtained, tan $\delta$ at 80° C and the blow-out temperature were shown in Table 13 below together with the compounding condition of the acrylamide and process oil.

Table 13

| Run No. | | Invention | | | | | |
|---|---|---|---|---|---|---|---|
| | | 1 | 2 | 3 | 4 | 5 | 6 |
| Compounding | Acrylamide | GA | GA | GA | GA | GB | GB |
| | Amount of acrylamide (parts) | 5 | 10 | 5 | 5 | 5 | 5 |
| | Amount of aromatic process oil (parts) | — | — | 10 | 20 | — | 20 |
| Result | Tan $\delta$ at 80°C | 0.194 | 0.197 | 0.196 | 0.202 | 0.183 | 0.187 |
| | Blow-out temperature (°C) | 280 | 280 | 270 | 270 | 280 | 270 |

(to be cont'd)

Table 13 (cont'd)

| Invention | | | | | | Comparison | | |
|---|---|---|---|---|---|---|---|---|
| 7 | 8 | 9 | 10 | 11 | 12 | 13 | 14 | 15 |
| GC | GC | GD | GE | GE | GF | — | — | — |
| 5 | 5 | 5 | 5 | 5 | 5 | — | — | — |
| — | 20 | — | — | 20 | — | — | 10 | 20 |
| 0.176 | 0.181 | 0.180 | 0.174 | 0.180 | 0.167 | 0.138 | 0.147 | 0.155 |
| 280 | 270 | 270 | 260 | 270 | 260 | 280 | 270 | 270 |

Example 14

| [Compounding formulation] | |
|---|---|
| Styrene/butadiene copolymer rubber (having a styrene content of 35 % and containing 37.5 parts of aromatic oil per 100 parts of the rubber) | shown in Table 14 |
| Natural rubber | shown in Table 14 |
| Butadiene rubber (BR-01) | shown in Table 14 |
| SAF carbon black | 65 parts |
| Stearic acid | 1 part |
| Zinc oxide | 3 parts |
| Aromatic process oil | 40 parts |
| Antioxidant (the same as that in Example 13) | 1 part |
| Vulcanization accelerator (the same as that in Example 13) | 1 part |
| Sulfur | 2 parts |
| Acrylamide | shown in Table 14 |

Vulcanized rubber was prepared based on the above compounding formulation and in concordance with the manner in Example 13, and it was subjected to the same tests as in Example 13. The results obtained were shown in Table 14 below together with the compounding condition of the base rubber and acrylamide.

Table 14

| | Run No. | Invention | | | | | | Comparison | | |
|---|---|---|---|---|---|---|---|---|---|---|
| | | 1 | 2 | 3 | 4 | 5 | 6 | 7 | 8 | 9 |
| Compounding | Styrene/butadiene copolymer rubber (parts) | 137.5 | 137.5 | 137.5 | 137.5 | — | — | 137.5 | — | — |
| | Natural rubber (parts) | — | — | — | — | 100 | 70 | — | 100 | 70 |
| | Butadiene rubber (parts) | — | — | — | — | — | 30 | — | — | 30 |
| | Acrylamide | GA | GB | GC | GD | GA | GA | — | — | — |
| | Amount of acrylamide (parts) | 5 | 5 | 5 | 5 | 5 | 5 | — | — | — |
| Result | Tan $\delta$ at 80°C | 0.232 | 0.227 | 0.199 | 0.189 | 0.174 | 0.173 | 0.181 | 0.144 | 0.149 |
| | Blow-out temperature (°C) | 270 | 260 | 260 | 250 | 190 | 210 | 270 | 190 | 220 |

The phenoxyaniline compound represented by the above formula (IV), one of the starting materials for the acrylamide compounds of the formula (III), can be prepared, for example, by reducing the aromatic nitro compound of the above formula (IVa) in an aqueous hydrochloric acid solution using bin (II) chloride as a catalyst, and the aromatic nitro compound (IVa) can be obtained by the condensation reaction between the phenolic compound of the above formula (IVb) and the nitrohalobenzene of the above formula (IVc) under a powdery copper catalyst. A typical preparation example of these starting compounds will be illustrated.

Preparation example of starting compound

Into a flask were charged 39.6 g (0.3 mole) of potassium phenolate, 49.4 g (0.35 mole) of 4-fluoro-1-nitrobenzene and 1.20 g of a powdery copper catalyst, and the mixture was kept warm at 200 °C for 2 hours. After completion of the reaction, the reaction mixture was cooled to 5 °C, and the precipitated crystals were collected by filtration, washed with 50 ml of cold diethyl ether, and then dried under a reduced pressure to obtain 52.6 g of 4-nitro-diphenyl ether. The melting point of the compound was 53° - 54 °C.

The whole of this compound was again charged into a flask, thereto was added 50 ml of glacial acetic acid, 3.0 g of tin (II) chloride and 400 ml of a 30 % aqueous hydrochloric acid solution, and the mixture was kept warm under a reflux condition for 5 hours. After completion of the reaction, the reaction mixture was cooled to 5 °C, and extracted with 100 ml of water. Further, the mixture was neutralized with a 33 % aqueous sodium hydroxide solution, washed with 100 ml of water, condensed under a reduced pressure, and thereafter recrystallized with hexane/ethyl acetate to obtain 31.2 g of 4-phenoxyaniline as pale yellow crystals.

This compound had a melting point of 82° - 84 °C, and showed the following elemental analysis result:

| | C | H | N |
|---|---|---|---|
| Found: | 77.75 % | 5.87 % | 7.63 % |
| Calculated: | 77.81 % | 5.94 % | 7.56 % |

Example 15

Into a flask were charged 90.8 g (1 mole) of acrylic acid and 473 g (4 mole) of thionyl chloride, and the mixture was kept warm under a reflux condition for 4 hours. After completion of the warming, the reaction mixture was cooled to a room temperature, then distilled to obtain 45.8 g of acryloyl chloride. Its boiling point was 72° - 74° C.

On the other hand, into a flask was charged 18.5 g (0.1 mole) of 4-phenoxyaniline, 13.1 g (0.13 mole) of triethylamine and 400 ml of chloroform, and thereto was added dropwise 10.9 g (0.12 mole) of acryloyl chloride at a temperature of 25° - 35 °C over a period of 30 minutes. Successibly, the reaction mixture was kept warm at 35 °C for 2 hours.

After completion of the reaction, the reaction mixture was extracted by pouring thereto 200 ml of water. The organic layer was washed with 200 ml of water, and then the solvent was distilled off. The precipitated crystals were washed with 30 ml of ether and then dryed under a reduced pressure to obtain 23.0 g of a product. The product was in a state of white crystals, and had a melting point of 114° -115 °C.

This compound, above-mentioned HA, was confirmed from an elemental analysis result and identification by NMR spectra as being the following structural formula:

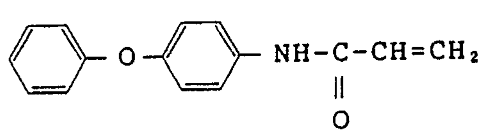

| Elemental analysis | | | |
|---|---|---|---|
| | C | H | N |
| Found: | 75.25 % | 5.48 % | 5.81 % |
| Calculated: | 75.33 % | 5.44 % | 5.85 % |

In accordance with the above manner but replacing the starting material, the above-mentioned compounds HB through HE were prepared.

Example 16

| [Compounding formulation] | |
|---|---|
| Styrene/butadiene copolymer rubber (styrene content of 25 %) | 100 parts |
| ISAF carbon black | 50 parts |
| Stearic acid | 3 parts |
| Zinc oxide | 5 parts |
| Aromatic process oil | shown in Table 15 |
| Antioxidant (N-Phenyl-N′-1,3-dimethylbutyl-p-phenylenediamine) | 1 part |
| Vulcanization accelerator (N-Cyclohexyl-2-benzothiazylsulfenamide) | 1 part |
| Sulfur | 2 parts |
| Acrylamide (HA through HE) | shown in Table 15 |

Using 250 ml Laboplastomill® manufactured by Toyo Seiki Co. as a Bumbury's mixer, and at an oil bath temperature of 170° C, thereto was charged the basal styrene/butadiene copolymer rubber, subjective acrylamide of the present invention, carbon black, stearic acid, process oil, antioxidant and zinc oxide in accordance with the above compounding formulation, and the mixture was kneaded for 5 minutes with a mixer revolution of 60 rpm. The rubber temperature at the kneading was 160° - 170° C.

The rubber blend was then transferred to an open mill, and thereto was added the vulcanization accelerator and sulfur shown in the above formulation at a temperature of 40° - 50° C, followed by kneading.

The kneaded mixture was then vulcanized at 150° C for 50 minutes using a vulcanizing press to form a predetermined shape, and the shaped vulcanized composition was subjected to the measurement of tan δ. Tan δ was determined using a viscoelasticity spectrometer manufactured by Iwamoto Seisakusho Co. under a frequency of 10 Hz and at respective temperatures of from a room temperature to 100° C.

A heat resistance test was also conducted by measuring a blow-out temperature for a vulcanized rubber specimen of about 1 cm x 1 cm x 3.5 mm which was prepared from the above rubber composition. Thus, the vulcanized rubber specimen was put in a thermostat, and the blow-out temperature was determined by such a method that while elevating the temperature from 200° C to 300° C at regular intervals of 10° C, the specimen was left at each temperature for 1 hour, thereafter was taken out and cut in half to visually observe whether bubbles were grown inside the specimen. A temperature at which the bubbles were observed for the first time was determined as the blow-out temperature.

Of the test results obtained, tan δ at 80° C and the blow-out temperature were shown in Table 15 below together with the compounding condition of the acrylamide and process oil.

Table 15

| | Run No. | Invention | | | | | |
|---|---|---|---|---|---|---|---|
| | | 1 | 2 | 3 | 4 | 5 | 6 |
| Compounding | Acrylamide | HA | HA | HA | HA | HA | HB |
| | Amount of acrylamide (parts) | 2 | 5 | 10 | 2 | 2 | 2 |
| | Amount of aromatic process oil (parts) | - | - | - | 10 | 20 | - |
| Result | Tan $\delta$ at 80°C | 0.175 | 0.196 | 0.201 | 0.184 | 0.193 | 0.174 |
| | Blow-out temperature (°C) | 280 | 280 | 270 | 280 | 270 | 270 |

(to be cont'd)

Table 15 (cont'd)

| Invention | | | Comparison | | |
|---|---|---|---|---|---|
| 7 | 8 | 9 | 10 | 11 | 12 |
| HC | HD | HE | - | - | - |
| 2 | 2 | 2 | - | - | - |
| - | - | - | - | 10 | 20 |
| 0.167 | 0.177 | 0.165 | 0.141 | 0.149 | 0.159 |
| 280 | 280 | 270 | 280 | 280 | 270 |

Example 17

| [Compounding formulation] | |
|---|---|
| Styrene/butadiene copolymer rubber (Nipol # 9520 produced by Nippon Zeon Co. having a styrene content of 35 % and containing 37.5 parts of aromatic oil per 100 parts of the rubber) | shown in Table 16 |
| Natural rubber | shown in Table 16 |
| Butadiene rubber (BR-01) | shown in Table 16 |
| SAF carbon black | 65 parts |
| Stearic acid | 1 part |
| Zinc oxide | 3 parts |
| Aromatic process oil | 40 parts |
| Antioxidant (the same as that in Example 16) | 1 part |
| Vulcanization accelerator (the same as that in Example 16) | 1 part |
| Sulfur | 2 parts |
| Acrylamide | shown in Table 16 |

Vulcanized rubber was prepared based on the above compounding formulation and in concordance with

the manner in Example 16, and it was subjected to the same tests as in Example 16. The results obtained were shown in Table 16 below together with the compounding condition of the base rubber and the acrylamide.

Table 16

| Run No. | | Invention | | | |
|---|---|---|---|---|---|
| | | 1 | 2 | 3 | 4 |
| Compounding | Styrene/butadiene copolymer rubber (parts) | 137.5 | 137.5 | 137.5 | 137.5 |
| | Natural rubber (parts) | - | - | - | - |
| | Butadiene rubber (parts) | - | - | - | - |
| | Acrylamide | HA | HA | HA | HB |
| | Amount of acrylamide (parts) | 2 | 5 | 10 | 2 |
| Result | Tan δ at 80°C | 0.221 | 0.239 | 0.244 | 0.218 |
| | Blow-out temperature (°C) | 270 | 260 | 260 | 270 |

(to be cont'd)

Table 16 (cont'd)

| Invention | | | Comparison | | |
|---|---|---|---|---|---|
| 5 | 6 | 7 | 8 | 9 | 10 |
| 137.5 | - | - | 137.5 | - | - |
| - | 100 | 70 | - | 100 | 70 |
| - | - | 30 | - | - | 30 |
| HD | HA | HA | - | - | - |
| 2 | 2 | 2 | - | - | - |
| 0.224 | 0.170 | 0.176 | 0.183 | 0.142 | 0.147 |
| 260 | 190 | 220 | 270 | 190 | 220 |

## Claims

1. A rubber composition arising natural for synthetic rubber, filler and acrylamide compound of formula

$$X - NH\underset{\underset{\displaystyle O}{\|}}{C}CH = CH_2$$

wherein X is an aliphatic, alicyclic, phenyl or phenyoxyphenyl group, in which said aliphatic or alicyclic

group may contain halogen or oxygen in the group, said phenyl nay be substituted once or twice by substituent(s) selected halogen, hydroxy, alkyl of 1 to 8 carbon atoms, of alkoxy 1 to 8 carbon atoms, nitro, cyano, carboxy and alkoxycarbonyl, and said phenoxyphenyl is of formula

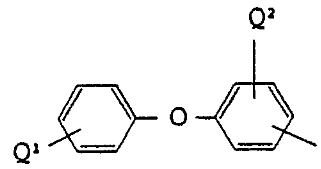

wherein $Q^1$ and $Q^2$ are selected independently from hydrogen, alkyl of 1 to 8 carbon atoms, cyclohexyl, phenyl, halogen, hydroxy and amino.

2. A rubber composition according to claim 1 wherein the filler is carbon black having a nitrogen absorption specific surface area of 80 to 250 $m^2/g$.

3. A rubber composition according to claim 1 or 2 wherein X is an aliphatic group of 1 to 20 carbon atoms, preferably a branched aliphatic group of 3 to 20 carbon atoms.

4. A rubber composition according to claim 1 or 2 or 3 wherein the acrylamide compound is selected from [a] N-phenylacrylamide; [b] compounds of formula

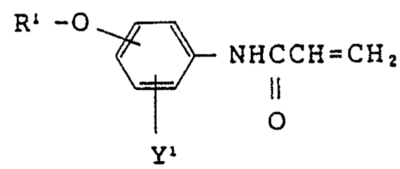

in which $R^1$ is hydrogen or alkyl, and $Y^1$ is hydrogen, halogen, hydroxy, alkoxy, cyano, carboxy or alkoxycarbonyl (and preferably wherein $R^1$-O- is attached at 4-position of the benzene ring and $Y^1$ is hydrogen); [c] compounds of formula

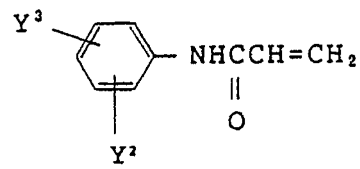

in which one of $Y^2$ and $Y^3$ is halogen, and the other is hydrogen, halogen, cyano, carboxy or alkoxycarbonyl (and preferably wherein one of $Y^2$ and $Y^3$ is halogen attached at 3-or 4-position of the benzene ring and the other is hydrogen; [d] compounds of formula

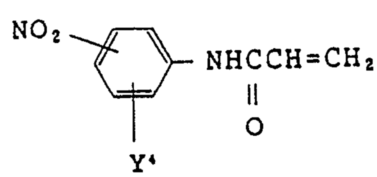

in which $Y^4$ is hydrogen, alkyl, alkoxy, halogen, nitro, cyano hydroxy, carboxy or alkoxycarbonyl (and preferably wherein $NO_2$- is attached at 3- or 4-position of the benzene ring, and $Y^4$ is hydrogen, alkoxy or nitro); [e] compounds of formula

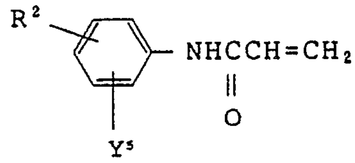

in which $R^2$ is alkyl, and $Y^5$ is hydrogen, alkyl, halogen, hydroxy, alkoxy, cyano, carboxy or alkoxycarbonyl (and preferably wherein $R^2$ is methyl or ethyl attached at 4-position of the benzene ring and $Y^5$ is hydrogen); [f] compounds of formula

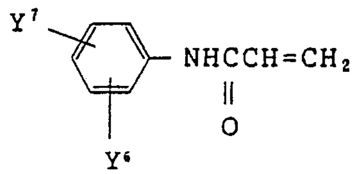

in which one of $Y^6$ and $Y^7$ is cyano,carboxy or alkoxycarbonyl (preferably N-(4-cyanophenyl)acrylamide); and [g] compounds of formula

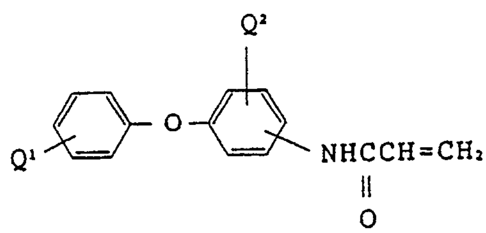

wherein $Q^1$ is hydrogen, alkyl of 1 to 4 carbon atoms, chlorine, or amino unsubstituted or substituted by alkyl of 1 to 4 carbon atoms, and $Q^2$ is hydrogen or alkyl of 1 to 4 carbon atoms.

5. A rubber composition according to any preceding claim wherein the rubber comprises styrene/butadiene copolymer rubber having a styrene content of 20 to 50 % by weight.

6. A rubber composition according to any preceding claim which further includes process oil.

7. A method for increasing the loss factor of a rubber which comprises blending the rubber with filler and acrylamide compound of formula

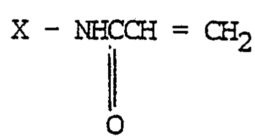

wherein X is an aliphatic, alicyclic, phenyl or phenoxyphenyl group, in which said aliphatic or alicyclic group may contain halogen or oxygen in the group, said phenyl may be substituted once or twice by substituent(s) selected from halogen, hydroxy, alkyl of 1 to 8 carbon atoms, alkoxy of 1 to 8 carbon atoms, nitro, cyano, carboxy and alkoxycarbonyl, and said phenoxyphenyl is of formula

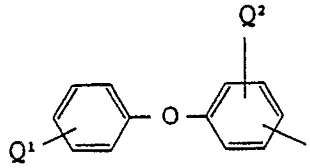

wherein $Q^1$ and $Q^2$ are selected independently from hydrogen, alkyl of 1 to 8 carbon atoms, cyclohexyl, phenyl, halogen, hydroxy and amino, said X preferably being an aliphatic group of 1 to 20 carbon atoms, cyclohexyl, or unsubstituted or substituted phenyl.

8. An acrylamide compound of formula

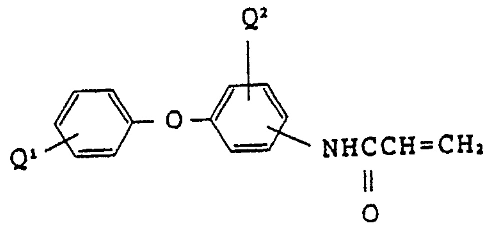

wherein $Q^1$ and $Q^2$ are selected independently from hydrogen, alkyl of 1 to 8 carbon atoms, cyclohexyl, phenyl, halogen, hydroxy and amino, preferably wherein [a] $Q^1$ and $Q^2$ are selected independently from hydrogen, alkyl of 1 to 4 carbon atoms, chlorine, and amino unsubstituted or substituted by alkyl of 1 to 4 carbon atoms, phenyl or cyclohexyl, or [b] $Q^1$ is hydrogen, alkyl of 1 to 4 carbon atoms, chlorine, or unsubstituted or substituted by alkyl of 1 to 4 carbon atoms, and $Q^2$ is hydrogen or alkyl of 1 to 4 carbon atoms.

9. A compound according to claim 8 which is 4- or 3-phenoxyphenylacrylamide.

10. A method for producing an acrylamide compound according to claim 8 which comprises subjecting phenoxyaniline compound of formula

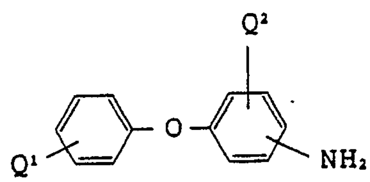

(wherein $Q^1$ and $Q^2$ are as defined in claim 8) to a condensation reaction with acryloyl halide in an inert solvent in the presence of dehydrohalogenating agent.

European Patent Office

# EUROPEAN SEARCH REPORT

Application Number

**EP 90 30 7807**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | DE-A-2 643 919 (GOODYEAR TIRE & RUBBER)<br>* Claims 1-3; page 7 *<br>– – – | 1-7 | C 08 K 5/20<br>C 08 L 21/00 |
| A | FR-A-2 256 940 (AMERICAN CYANAMID)<br>* Claim 1 *<br>– – – | 1-7 | |
| X | DATABASE CHEMICAL ABSTRACTS, (HOST:STN), vol. 102, no. 2, 1984, abstract no. 7616p, Columbus, Ohio, US; & JP-A-59 108 031 (UBE INDUSTRIES) 22-06-1982<br>* Abstract, line 10 *<br>– – – | 8 | |
| A | US-A-3 976 470 (STAUFFER CHEMICAL)<br>* Column 1, lines 10-27; column 2, lines 16-34 *<br>– – – | 8,10 | |
| A | DATABASE WPIL, accession no. 87-338153 [48], Derwent Publications Ltd, London, GB; & JP-A-62 242 654 (AGENCY OF IND. SCI. TECH.) 23-10-1987<br>* Abstract *<br>– – – – – | 10 | |
| | | | **TECHNICAL FIELDS SEARCHED (Int. Cl.5)** |
| | | | C 08 K<br>C 08 L |

The present search report has been drawn up for all claims

| Place of search | Date of completion of search | Examiner |
|---|---|---|
| The Hague | 07 November 90 | VAN HUMBEECK F.W.C. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document